# EUROPÄISCHE PATENTANMELDUNG

(11) **EP 0 937 723 A1**
(43) Veröffentlichungstag der Anmeldung: **25.08.1999**
(21) Anmeldenummer: 98102750.1
(22) Anmeldetag: 18.02.1998
(51) Int. Cl.: C07D 401/12, C07D 409/14, A61K 31/47

(54) **Neue Sulfonamide, Verfahren zu ihrer Herstellung sowie diese enthaltende Arzneimittel**

(71) Anmelder: Roche Diagnostics GmbH, 68298 Mannheim (DE)
(72) Erfinder: Kucznierz, Ralf, Dr., 67098 Bad Dürkheim (DE); Grams, Frank, Dr., 68219 Mannheim (DE); Leinert, Herbert, Dr., 64646 Heppenheim (DE); von der Saal, Wolfgang, Dr., 69469 Weinheim (DE); Stegmeier, Karlheinz, Dr., 64646 Heppenheim (DE)

(57) **Zusammenfassung**

Die Erfindung betrifft neue Sulfonamide der allgemeinen Formel I 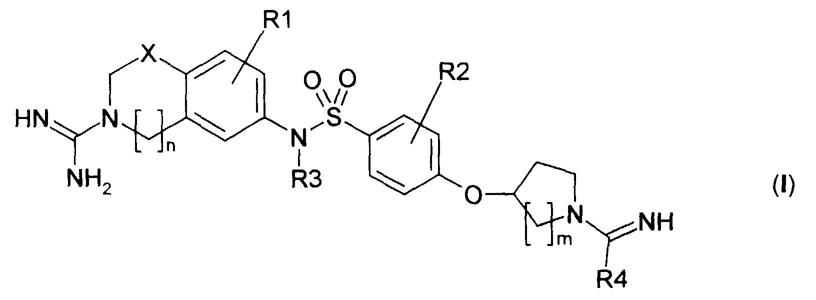 in der R¹ bis R⁴ die oben angegebene Bedeutung besitzen, sowie deren Hydrate, Solvate und physiologisch verträgliche Salze, optisch aktive Formen, Racemate und Diastereomerengemische, Verfahren zu ihrer Herstellung sowie Arzneimittel, die diese Verbindungen enthalten, zur Behandlung thromboembolischer Erkrankungen..

## Beschreibung

Die Erfindung betrifft neue Sulfonamide der allgemeinen Formel I 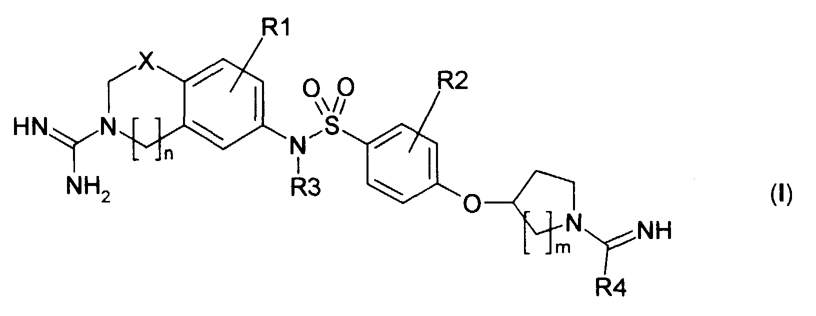 in der
- R¹, R²: unabhängig voneinander ein Wasserstoffatom, ein Halogenatom, eine Hydroxygruppe, eine Alkylgruppe, eine Cycloalkylgruppe, eine Alkenylgruppe, eine Alkinylgruppe, ein Arylrest, ein Heteroarylrest, eine Alkoxygruppe, eine Aralkyloxygruppe, eine Alkenyloxygruppe, eine Alkinyloxygruppe, eine Carboxylgruppe, eine Alkoxycarbonylgruppe, eine Alkenyloxycarbonylgruppe, eine Alkinyloxycarbonylgruppe, eine Hydroxyalkylgruppe, eine Alkoxyalkylgruppe, eine Carboxyalkylgruppe, eine Alkyloxycarbonylalkylgruppe, eine Alkenyloxycarbonylalkylgruppe oder eine Alkinyloxycarbonylalkylgruppe sein können;
- R³: ein Wasserstoffatom, eine Alkylgruppe, eine Cycloalkylgruppe, eine Alkenylgruppe, eine Alkinylgruppe, einen Aralkylrest, eine Hydroxyalkylgruppe, eine Alkoxyalkylgruppe, eine Aminoalkylgruppe, eine Carboxyalkylgruppe, eine Alkyloxycarbonylalkylgruppe, eine Alkenyloxycarbonylalkylgruppe oder eine Alkinyloxycarbonylalkylgruppe, ein Alkylcarbonylrest, eine Arylcarbonylgruppe, eine Carboxyalkylsulfonylgruppe, eine Alkyloxycarbonylalkylsulfonylgruppe, eine Dihydroxyborylalkylgruppe, eine Dialkoxyborylalkylgruppe oder eine gegebenenfalls substituierte 1,3,2-Dioxaborolanylalkylgruppe oder eine gegebenenfalls substituierte 1,3,2-Dioxaborinanylalkylgruppe sein kann;
- R⁴: eine gegebenenfalls substituierte Aminogruppe, eine Alkylgruppe, einen Cycloalkylrest, einen gegebenenfalls substituierten Arylrest oder einen gegebenenfalls substituierten Heteroarylrest bedeutet;
- X: eine Einfachbindung, eine Carbonylgruppe eine Alkylen- oder eine Alkylenoxygruppe bedeutet;
- n: die Zahl 1 oder 2 bedeutet und
- m: eine ganze Zahl zwischen 1 und 4 bedeutet,
sowie Hydrate, Solvate und physiologisch verträgliche Salze davon. Gegenstand der Enfindung sind auch die optisch aktiven Formen, die Racemate und die Diastereomerengemische dieser Verbindungen.

Die Erfindung betrifft auch Verfahren zur Herstellung der obigen Verbindungen, Arzneimittel, die solche Verbindungen enthalten, sowie die Verwendung dieser Verbindungen bei der Herstellung von Arzneimitteln.

Die Sulfonamide der allgemeinen Formel I, ihre Solvate und ihre Salze greifen durch reversible Inhibierung von Faktor Xa in den Prozeß der Blutgerinnung ein und verhindern somit die Entstehung von Gerinnungsthromben. Sie können deshalb bei der Bekämpfung und Verhütung von Krankheiten, wie Thrombose, Apoplexie, Herzinfarkt, Entzündungen und Arteriosklerose, verwendet werden.

Faktor Xa ist eine Serinprotease des Gerinnungssystems, die die proteolytische Umwandlung von Prothrombin in Thrombin katalysiert. Thrombin, als letztes Enzym der Gerinnungskaskade, spaltet einerseits Fibrinogen zu Fibrin, das nach Quervenetzung mittels Faktor XIIIa zu einem unlöslichen Gel wird und die Matrix für einen Thrombus bildet, andererseits aktiviert es durch Proteolyse seines Rezeptors auf den Blutplättchen die Plättchenaggregation und trägt auf diesem Weg ebenfalls zur Thrombusbildung bei. Bei der Verletzung eines Blutgefäßes sind diese Prozesse notwendig, um eine Blutung zu stoppen. Unter normalen Umständen sind keine meßbaren Thrombin-Konzentrationen im Blutplasma vorhanden. Ein Ansteigen der Thrombinkonzentration kann zur Ausbildung von Thromben und damit zu thromboembolischen Krankheiten führen, die vor allem in den Industriestaaten sehr häufig auftreten. Durch Hemmung von Faktor Xa kann die Entstehung von Thrombin verhindert werden.

Kürzlich wurde berichtet, daß Amidinoarylpropansäure-Derivate wie (+)-(2S)-2-[4-[[(3S)-1-Acetimidoyl-3-pyrrolidinyl]oxy]phenyl]-3-(7-amidino-2-naphthyl]propansäure-hydrochlorid-pentahydrat (DX-9065a; Formel IIa) Faktor Xa inhibieren (*J. Med. Chem*. **1994**, *37*, 1200-1207; *Thrombosis and Haemostasis* **1994**, *71*, 314-319; EP-0-540-051-A-1). Weitere bekannte Faktor-Xa-Inhibitoren sind 1,2-Bis-(5-amidino-2-benzofuranyl)-ethan (DABE, Formel IIb, *Thrombosis Research* **1980**, *19*, 339-349) oder auch Phenylamino-methyl-naphthamidine der allgemeinen Formel IIc (WO96/16940).

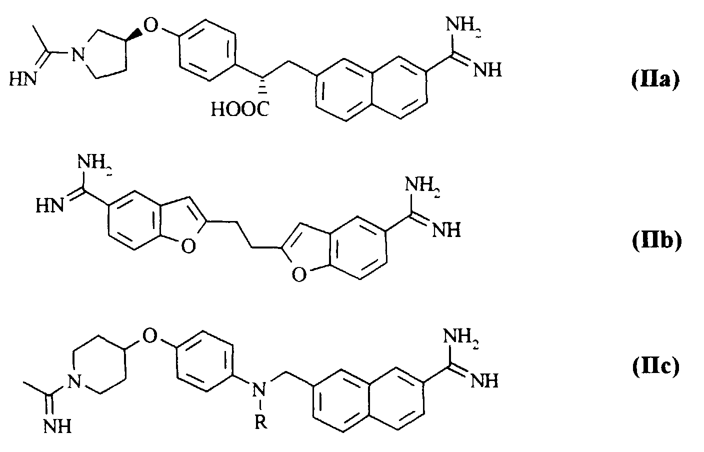

Die erfindungsgemäßen, neuen Sulfonamide der allgemeinen Formel I sowie Hydrate, Solvate und physiologisch verträgliche Salze davon sind potente und selektive Faktor Xa-Inhibitoren.

In der allgemeinen Formel I können die Substituenten R¹ und R² gleich oder verschiedenartig sein

Bedeutet R¹, R² in der allgemeinen Formel I ein Halogenatom, so kann dies jeweils ein Fluor-, Chlor-, Brom- oder Iodatom bedeuten, bevorzugt sind jedoch Fluor-, Chlor- oder Bromsubstituenten.

Bedeutet R¹, R², R³, R⁴ in der allgemeinen Formel I eine Alkylgruppe, so kann diese geradkettig oder verzweigt sein und 1 bis 8 Kohlenstoffatome enthalten. Bevorzugt sind die Methyl-, Ethyl-, *n*-Propyl-, *i*-Propyl-, *n*-Butyl-, *i*-Butyl-, *t*-Butyl-, Pentyl- und die Hexylgruppe.

Bedeutet R¹, R², R³, R⁴ in der allgemeinen Formel I eine Cycloalkylgruppe, so kann diese substituiert oder unsubstituiert sein und 3 bis 8 Kohlenstoffatome enthalten. Bevorzugt sind die Cyclopropyl-, Cyclopentyl-, Cyclohexyl- und die Cyclooctylgruppe.

Bedeutet R¹, R², R³ in der allgemeinen Formel I eine Alkenylgruppe, so kann diese geradkettig oder verzweigt sein und 2 bis 8 Kohlenstoffatome enthalten. Bevorzugt sind die Vinyl-, 1-Propenyl-, 2-Propenyl-, 2-Methyl-2-propenyl-, 1-Butenyl-, 1-Pentenyl- und die 1-Hexenylgruppe.

Bedeutet R¹, R² in der allgemeinen Formel I eine Alkinylgruppe, so kann diese geradkettig oder verzweigt sein und 2 bis 8 Kohlenstoffatome enthalten. Bevorzugt sind die Ethinyl- und die Propargylgruppe.

Bedeutet R¹, R², R⁴ in der allgemeinen Formel I einen Arylrest, so versteht man darunter die Phenyl-, eine Biphenyl- oder eine Naphthylgruppe. Der Arylrest kann unsubstituiert sein oder gegebenenfalls einen oder mehrere C₁-C₈-Alkylsubstituenten, vorzugsweise Methyl, einen oder mehrere C₁-C₈-Alkyloxysubstituenten, vorzugsweise Methoxy, eine oder mehrere Carboxylgruppen, einen oder mehrere C₁-C₈-Alkoxycarbonylsubstituenten, vorzugsweise Methoxycarbonyl oder Ethoxycarbonyl, oder einen oder mehrere Halogensubstituenten tragen. Die Spezifizierung C₁-C₈ steht hier jeweils für eine geradkettige oder verzweigte Alkylkette mit 1 bis 8 Kohlenstoffatomen. Halogene als Substituenten des Arylrestes können Fluor-, Chlor-, Brom- und Iodatome, bevorzugt jedoch Fluor-, Chlor- oder Bromatome sein.

Bedeutet R¹, R², R⁴ in der allgemeinen Formel I einen Heteroarylrest, so versteht man darunter einen Thiophenyl-, einen Benzothiophenyl-, einen Furanyl-, einen Benzofuranyl-, einen Chinolinyl-, oder einen Isochinolinylrest. Der Heteroarylrest kann unsubstituiert sein oder gegebenenfalls einen oder mehrere C₁-C₈-Alkylsubstituenten, vorzugsweise Methyl, einen oder mehrere C₁-C₈-Alkyloxysubstituenten, vorzugsweise Methoxy, eine oder mehrere Carboxylgruppen, einen oder mehrere C₁-C₈-Alkoxycarbonylsubstituenten, vorzugsweise Methoxycarbonyl oder Ethoxycarbonyl, oder einen oder mehrere Halogensubstituenten tragen. Die Spezifizierung C₁-C₈ steht hier jeweils für eine geradkettige oder verzweigte Alkylkette mit 1 bis 8 Kohlenstoffatomen. Halogene als Substituenten des Heteroarylrestes können Fluor-, Chlor-, Brom- und Iodatome, bevorzugt jedoch Fluor-, Chlor- oder Bromatome sein.

Alkoxygruppen als Substituenten R¹, R² in der allgemeinen Formel I enthalten 1 bis 8 Kohlenstoffatome und sind geradkettig oder verzweigt. Bevorzugt sind die Methoxy-, Ethoxy-, *n*-Propyloxy-, *i*-Propyloxy-, *n*-Butyloxy-, *i*-Butyloxy-, *tert*.-Butyloxy-, Pentyloxy- und die Hexyloxygruppe.

Bedeutet R¹, R² in der allgemeinen Formel I eine Aralkyloxygruppe, so enthält diese eine mit einer geradkettigen oder verzweigten C₁-C₈-Alkylkette verknüpfte Phenylgruppe, eine mit einer geradkettigen oder verzweigten C₁-C₈-Alkylkette verknüpfte Naphthylgruppe oder eine mit einer geradkettigen oder verzweigten C₁-C₈-Alkylkette verknüpfte Biphenylgruppe. Bevorzugt sind dabei die Benzyloxygruppe, die p-Phenylbenzyloxygruppe und die Naphthylmethyloxygruppe.

Alkenyloxygruppen als Substituenten R¹, R² in der allgemeinen Formel I enthalten 3 bis 8 Kohlenstoffatome und sind geradkettig oder verzweigt. Bevorzugt sind die Vinyloxy- und Allyloxygruppe.

Alkinyloxygruppen als Substituenten R¹, R² in der allgemeinen Formel I enthalten 3 bis 8 Kohlenstoffatome und sind geradkettig oder verzweigt. Bevorzugt ist die Propargyloxygruppe.

Alkoxycarbonylgruppen als Substituenten R¹, R² in der allgemeinen Formel I enthalten geradkettige oder verzweigte Alkylketten mit 1 bis 8 Kohlenstoffatomen. Bevorzugt sind die Methoxycarbonyl- und die Ethoxycarbonylgruppe sowie die *i*-Propyloxycarbonyl- und die *tert*.-Butyloxycarbonylgruppe.

Bedeutet R¹, R² in der allgemeinen Formel I eine Alkenyloxycarbonylgruppe, so enthält diese geradkettige oder verzweigte Alkenyle mit 3 bis 8 Kohlenstoffatomen. Bevorzugt ist die Allyloxycarbonylgruppe.

Bedeutet R¹, R² in der allgemeinen Formel I eine Alkinyloxycarbonylgruppe, so enthält diese geradkettige oder verzweigte Alkinyle mit 3 bis 8 Kohlenstoffatomen. Bevorzugt ist die Propargyloxycarbonylgruppe.

Bedeutet R¹, R² in der allgemeinen Formel I eine Hydroxyalkylgruppe, so kann diese geradkettig oder verzweigt sein und 1 bis 8 Kohlenstoffatome enthalten. Bevorzugt sind die Hydroxymethyl-, Hydroxyethyl-, Hydroxypropyl-, Hydroxybutyl-, Hydroxypentyl- und die Hydroxyhexylgruppe.

Bedeutet R¹, R², R³ in der allgemeinen Formel I eine Alkoxyalkylgruppe, so sind unter den betreffenden Alkylresten jeweils geradkettige oder verzweigte Alkylketten mit 1 bis 8 Kohlenstoffatomen, zu verstehen. Bevorzugt sind die Methoxymethyl-, Ethoxymethyl-, Methoxyethyl- und die Ethoxyethylgruppe.

Carboxyalkylgruppen als Substituenten R¹, R² in der allgemeinen Formel I enthalten Alkylgruppen mit 1 bis 8 Kohlenstoffatomen und sind geradkettig oder verzweigt. Bevorzugt sind die Carboxymethyl-, die Carboxyethyl- sowie die Carboxypropylgruppe.

Bedeutet R¹, R², R³ in der allgemeinen Formel I eine Alkyloxycarbonylalkylgruppe, so sind unter den Alkylresten jeweils geradkettige oder verzweigte Alkylketten mit 1 bis 8 Kohlenstoffatomen, zu verstehen. Bevorzugt sind die Methoxycarbonylmethyl-, Ethoxycarbonylmethyl-, Methoxycarbonylethyl-, Ethoxycarbonylethyl-, Methoxycarbonylpropyl- und die Ethoxycarbonylpropylgruppe.

Bedeutet R¹, R², R³ in der allgemeinen Formel I eine Alkenyloxycarbonylalkylgruppe, so sind die Alkenylreste geradkettig oder verzweigt mit 3 bis 8 Kohlenstoffatomen und die Alkylgruppen geradkettig oder verzweigt mit 1 bis 8 Kohlenstoffatomen. Bevorzugt sind die Allyloxycarbonylmethyl-, Allyloxycarbonylethyl- und die Allyloxycarbonylpropylgruppe.

Bedeutet R¹, R², R³ in der allgemeinen Formel I eine Alkinyloxycarbonylalkylgruppe, so sind die Alkinylreste geradkettig oder verzweigt mit 3 bis 8 Kohlenstoffatomen und die Alkylgruppen geradkettig oder verzweigt mit 1 bis 8 Kohlenstoffatomen. Bevorzugt sind die Propargyloxycarbonylmethyl-, Propargyloxycarbonylethyl- und die Propargyloxycarbonylpropylgruppe.

Bedeutet R³ in der allgemeinen Formel I eine Alkinylgruppe, so kann diese geradkettig oder verzweigt sein und 3 bis 8 Kohlenstoffatome enthalten. Bevorzugt ist die Propargylgruppe.

Unter einem Aralkylrest als Substituent R³ in der allgemeinen Formel I versteht man eine mit einer geradkettigen oder verzweigten C₁-C₈-Alkylkette verknüpfte Phenylgruppe, eine mit einer geradkettigen oder verzweigten C₁-C₈-Alkylkette verknüpfte Naphthylgruppe oder eine mit einer geradkettigen oder verzweigten C₁-C₈-Alkylkette verknüpfte Biphenylgruppe. Bevorzugt sind dabei die Benzylgruppe, die p-Phenylbenzylgruppe und die Naphthylmethylgruppe.

Bedeutet R³ in der allgemeinen Formel I eine Hydroxyalkylgruppe, so kann diese geradkettig oder verzweigt sein und 2 bis 8 Kohlenstoffatome enthalten. Bevorzugt sind die Hydroxyethyl-, Hydroxypropyl-, Hydroxybutyl-, Hydroxypentyl- und die Hydroxyhexylgruppe.

Bedeutet R³ in der allgemeinen Formel I eine Aminoalkylgruppe, so kann diese geradkettig oder verzweigt sein und 2 bis 8 Kohlenstoffatome enthalten. Bevorzugt sind die Aminoethyl-, Aminopropyl-, Aminobutyl-, Aminopentyl- und die Aminohexylgruppe.

Eine Carboxyalkylgruppe als Substituent R³ in der allgemeinen Formel I enthält eine Alkylkette mit 1 bis 8 Kohlenstoffatomen und ist geradkettig oder verzweigt. Bevorzugt sind die Carboxymethyl-, die Carboxyethyl- sowie die Carboxypropylgruppe.

Bedeutet R³ in der allgemeinen Formel I einen Alkylcarbonylrest, so kann die Alkylgruppe geradkettig oder verzweigt sein und 1 bis 8 Kohlenstoffatome enthalten. Bevorzugt sind die Acetyl- und die Propionylgruppe.

Eine Arylcarbonylgruppe als Rest R³ in der allgemeinen Formel I enthält als Arylfragment eine Phenyl-, eine Biphenyl- oder gegebenenfalls eine Naphthylgruppe, bevorzugt jedoch eine Phenylgruppe. Der Arylrest kann unsubstituiert sein oder gegebenenfalls einen oder mehrere C₁-C₈-Alkylsubstituenten, vorzugsweise Methyl, einen oder mehrere C₁-C₈-Alkyloxysubstituenten, vorzugsweise Methoxy, eine oder mehrere Carboxylgruppen, einen oder mehrere C₁-C₈-Alkoxycarbonylsubstituenten, vorzugsweise Methoxycarbonyl oder Ethoxycarbonyl, oder einen oder mehrere Halogensubstituenten tragen. Die Spezifizierung C₁-C₈ steht hier jeweils für eine geradkettige oder verzweigte Alkylkette mit 1 bis 8 Kohlenstoffatomen. Halogene als Substituenten des Arylrestes können Fluor-, Chlor-, Brom- und Iodatome, bevorzugt jedoch Fluor-, Chlor- oder Bromatome sein.

Bedeutet R³ in der allgemeinen Formel I eine Carboxyalkylsulfonylgruppe so enthält diese eine Alkylkette mit 1 bis 8 Kohlenstoffatomen, die geradkettig oder auch verzweigt sein kann. Bevorzugt sind die Carboxymethylsulfonyl-, die Carboxyethylsulfonyl- sowie die Carboxypropylsulfonylgruppe.

Bedeutet R³ in der allgemeinen Formel I eine Alkyloxycarbonylalkylsulfonylgruppe, so sind unter den Alkylresten jeweils geradkettige oder verzweigte Alkylketten mit 1 bis 8 Kohlenstoffatomen zu verstehen. Bevorzugt sind die Methoxycarbonylmethylsulfonyl-, Ethoxycarbonylmethylsulfonyl-, Methoxycarbonylethylsulfonyl-, Ethoxycarbonylethylsulfonyl-, Methoxycarbonylpropylsulfonyl- und die Ethoxycarbonylpropylsulfonylgruppe.

Bedeutet R³ in der allgemeinen Formel I eine Dihydroxyborylalkylgruppe, so enthält diese eine geradkettige oder verzweigte Alkylkette mit 1 bis 8 Kohlenstoffatomen. Bevorzugt sind die Dihydroxyborylmethyl- und die Dihydroxyborylpropylgruppe.

Bedeutet R³ in der allgemeinen Formel I eine Dialkoxyborylalkylgruppe, so können die jeweiligen Alkylreste unabhängig voneinander geradkettig oder verzweigt sein und 1 bis 8 Kohlenstoffatome enthalten. Bevorzugt sind die Dimethoxyborylmethyl- und die Dimethoxyborylpropylgruppe.

Bedeutet R³ in der allgemeinen Formel I eine 1,3,2-Dioxaborolanylalkylgruppe, so kann der Alkylrest geradkettig oder verzweigt sein und 1 bis 8 Kohlenstoffatome enthalten. Gegebenenfalls kann der 1,3,2-Dioxaborolanylrest in 4- und 5-Stellung substituiert sein, und zwar mit bis zu vier Methylgruppen. Bevorzugt sind die 1,3,2-Dioxaborolanylmethylgruppe und die 4,4,5,5-Tetramethyl-1,3,2-dioxaborolanylmethylgruppe.

Bedeutet R⁴ in der allgemeinen Formel I eine Aminogruppe, so kann diese unsubstituiert oder auch substituiert sein und zwar mit einer oder zwei C₁-C₈-Alkylgruppen, vorzugsweise Methyl oder Ethyl, mit einer oder zwei C₃-C₈-Cycloalkylgruppen, vorzugsweise Cyclopropyl, Cyclopentyl, Cyclohexyl oder Cyclooctyl, mit einer oder zwei C₂-C₈-Hydroxyalkylgruppen, vorzugsweise Hydroxyethyl oder Hydroxypropyl, mit einer oder zwei C₃-C₈-Alkenylgruppen, vorzugsweise Allyl, mit einer oder zwei C₃-C₈-Alkinylgruppen, vorzugsweise Propargyl oder mit einer oder zwei Aralkylgruppen, vorzugsweise Benzyl. Die Spezifizierung C₁-C₈-Alkyl bezeichnet hier eine geradkettige oder verzweigte Alkylkette mit 1 bis 8 Kohlenstoffatomen. C₃-C₈-Cycloalkyl bezeichnet hier eine verzweigte oder unverzweigte Cycloalkylgruppe mit 3 bis 8 Kohlenstoffatomen. C₂-C₈-Hydroxyalkyl bezeichnet hier eine geradkettige oder verzweigte Alkylkette mit 2 bis 8 Kohlenstoffatomen, die durch eine oder mehrere Hydroxygruppen substituiert sein kann. C₃-C₈-Alkenyl bedeutet hier eine geradkettige oder verzweigte ungesättigte Kette von 3 bis 8 Kohlenstoffatomen. C₃-C₈-Alkinyl bedeutet hier eine geradkettige oder verzweigte Kette von 3 bis 8 Kohlenstoffatomen. Aralkyl bedeutet hier eine mit einer geradkettigen oder verzweigten C₁-C₈-Alkylkette verknüpfte Phenylgruppe, eine mit einer geradkettigen oder verzweigten C₁-C₈-Alkylkette verknüpfte Naphthylgruppe oder eine mit einer geradkettigen oder verzweigten C₁-C₈-Alkylkette verknüpfte Biphenylgruppe.

Bedeutet X in der allgemeinen Formel I eine Alkylengruppe, so kann diese geradkettig oder verzweigt sein und 1 bis 8 Kohlenstoffatome enthalten. Bevorzugt sind die Methylengruppe und die Ethylengruppe.

Bedeutet X in der allgemeinen Formel I eine Alkylenoxygruppe, so kann diese geradkettig oder verzweigt sein und 1 bis 8 Kohlenstoffatome enthalten. Bevorzugt ist das Methylenoxyfragment. Besonders bevorzugt sind Verbindungen der allgemeinen Formel I, in denen
- R¹, R²: gleich oder verschieden sind und ein Wasserstoffatom, ein Fluoratom, ein Chloratom, eine Hydroxygruppe, eine Methylgruppe, eine Ethylgruppe eine Methoxygruppe, eine Benzyloxygruppe, eine Allyloxygruppe, eine Carboxylgruppe, eine Methoxy- oder Ethoxycarbonylgruppe, eine Hydroxymethyl- oder Hydroxyethylgruppe oder eine Carboxymethylgruppe bedeuten;
- R³: ein Wasserstoffatom, eine Methylgruppe, eine Ethylgruppe, eine Isopropylgruppe, eine Cyclopropylgruppe, eine Cyclohexylgruppe, eine Allylgruppe, eine Propargylgruppe, eine Benzylgruppe, eine Hydroxyethylgruppe, eine Hydroxypropylgruppe, eine Methoxyethylgruppe, eine Aminoethylgruppe, eine Carboxymethylgruppe, eine Carboxyethylgruppe, eine Carboxypropylgruppe, eine Ethoxycarbonylmethylgruppe, eine Ethoxycarbonylethylgruppe, eine Ethoxycarbonylpropylgruppe, eine Acetylgruppe, eine 4-Methoxybenzoylgruppe, eine Carboxymethylsulfonylgruppe, eine Carboxyethylsulfonylgruppe, eine Carboxypropylsulfonylgruppe, eine Ethoxycarbonylmethylsulfonylgruppe, eine Ethoxycarbonylethylsulfonylgruppe, eine Ethoxycarbonylpropylsulfonylgruppe, eine Dihydroxyborylmethylgruppe, eine Dihydroxyborylpropylgruppe, eine 4,4,5,5-Tetramethyl-1,3,2-dioxaborolanylmethylgruppe bedeutet;
- R⁴: eine Aminogruppe, eine Methylgruppe, eine Ethylgruppe, eine Cyclopropylgruppe, eine Cyclohexylgruppe, eine 4-Methoxyphenylgruppe oder eine Thienylgruppierung bedeutet;
- X: eine Methylengruppe bedeutet;
- n: die Zahl 1 bedeutet und
- m: die Zahl 1 oder die Zahl 2 sein kann.

Insbesonders bevorzugt sind auch Verbindungen, in denen R¹, R² und R³ Wasserstoff, R⁴ die Gruppe NH₂, X die Methylengruppe, n die Zahl 1 und m die Zahl 2 bedeuten.

Unter den physiologisch verträglichen Salzen der allgemeinen Formel I versteht man beispielsweise Formiate, Acetate, Caproate, Oleate, Lactate oder Salze von Carbonsäuren mit bis zu 18 Kohlenstoffatomen oder Salze von Dicarbonsäuren und Tricarbonsäuren wie Citrate, Malonate und Tartrate oder Alkansulfonate mit bis zu 10 Kohlenstoffatomen oder p-Toluolsulfonate oder Salicylate oder Trifluoracetate oder Salze von physiologisch verträglichen Mineralsäuren wie Salzsäure, Bromwasserstoffsäure Iodwasserstoffsäure, Schwefelsäure, Phosphorsäure. Die Verbindungen der Formel I mit einer oder zwei freien Säuregruppen am Phosphonatfragment können auch Salze mit physiologisch verträglichen Basen bilden. Beispiele solcher Salze sind Alkalimetall-, Erdalkalimetall-, Ammonium- und Alkylammoniumsalze, wie das Natrium-, Kalium-, Calcium- oder Tetramethylammoniumsalz.

Die Verbindungen der Formel I können solvatisiert, insbesondere hydratisiert sein. Die Hydratisierung kann im Zuge der Herstellung erfolgen oder allmählich als Folge hygroskopischer Eigenschaften einer zunächst wasserfreien Verbindung der Formel I auftreten

Gegenstand der Erfindung sind auch die optisch aktiven Formen, die Racemate und die Diastereomerengemische von Verbindungen der allgemeinen Formel I.

Zur Herstellung von Arzneimitteln werden die Substanzen der allgemeinen Formel I mit geeigneten pharmazeutischen Trägersubstanzen, Aroma-, Geschmacks- und Farbstoffen gemischt und beispielsweise als Tabletten oder Dragees ausgeformt oder unter Zugabe entsprechender Hilfsstoffe in Wasser oder Öl, z.B. in Olivenöl, suspendiert oder gelöst.

Die Substanzen der allgemeinen Formel I und ihre Salze können in flüssiger oder fester Form oral, enteral oder parenteral appliziert werden. Bevorzugt ist die orale Applikationsform. Als Injektionsmedium kommt vorzugsweise Wasser zur Anwendung, welches die bei Injektionslösungen üblichen Zusätze wie Stabilisierungsmittel, Lösungsvermittler oder Puffer enthält. Derartige Zusätze sind z.B. Tartrat- und Citratpuffer, Komplexbildner (wie Ethylendiamintetraessigsäure und deren untoxische Salze) und hochmolekulare Polymere wie flüssiges Polyethylenoxid zur Viskositätsregulierung. Feste Trägerstoffe sind z.B. Stärke, Lactose, Mannit, Methylcellulose, Talcum, hochdisperse Kieselsäuren, hochmolekulare Fettsäuren (wie Stearinsäure), tierische und pflanzliche Fette und feste hochmolekulare Polymere (wie Polyethylenglykole). Für orale Applikation geeignete Zubereitungen können gewünschtenfalls Geschmacks- und Süßstoffe enthalten.

Die Verbindungen werden üblicherweise in Mengen von 1-1500 mg pro Tag bezogen auf 75 kg Körpergewicht appliziert Bevorzugt ist es, 2-3 mal pro Tag 1-2 Tabletten mit einem Wirkstoffgehalt von 1-500 mg zu verabreichen. Die Tabletten können auch retardiert sein, wodurch nur noch einmal pro Tag 1-2 Tabletten mit 2-700 mg Wirkstoff gegeben werden müssen. Der Wirkstoff kann auch durch Injektion 1-8 mal pro Tag oder durch Dauerinfusion gegeben werden, wobei 5-2000 mg pro Tag normalerweise ausreichen.

Die Herstellung von Verbindungen der allgemeinen Formel I erfolgt nach an sich bekannten Methoden.

Die Verbindungen der allgemeinen Formel I werden hergestellt, indem man beispielsweise eine Verbindung der allgemeinen Formel III 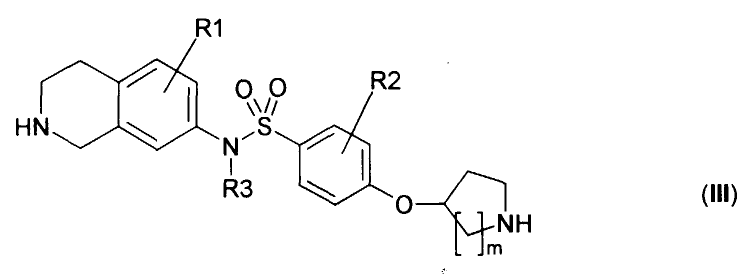 in der R¹, R², R³ und m die oben angegebenen Bedeutungen besitzen, mit einem Guanylierungsreagenz wie beispielsweise 1H-Pyrazol-1-carboxamidin (siehe z. B.: M. S. Bernatowicz, Y. Wu, G. R. Matsueda, *J. Org. Chem*. **199**2, *57*, 2497-2502) oder S-Methylisothioharnstoff in einem inerten Losungsmittel wie z. B. Dimethylformamid, Dioxan, Dimethylsulfoxid oder Toluol bei Temperaturen zwischen 0°C und Siedepunkt des Lösungsmittels, vorzugsweise bei 0 bis 30°C in Gegenwart einer Hilfsbase wie z. B Triethylamin, N-Methylmorpholin, Pyridin oder Ethyldiisopropylamin zur Reaktion bringt.

Die Verbindungen der allgemeinen Formel III werden hergestellt, indem man eine Verbindung der allgemeinen Formel IV 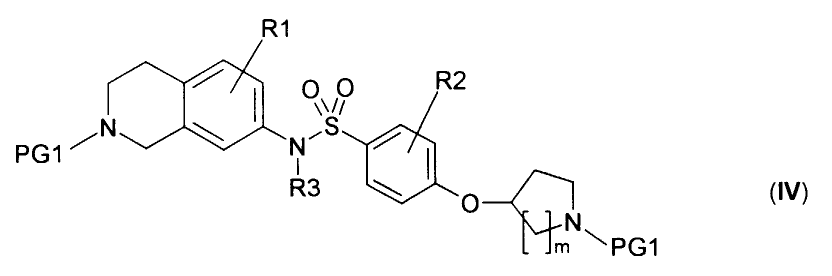 in der in der R¹, R², R³ und m die oben angegebenen Bedeutungen besitzen und PG¹ eine Schutzgruppe wie z. B. die Benzyloxycarbonylgruppe, die t-Butyloxycarbonylgruppe oder die Allyloxycarbonylgruppe bedeutet, mit einem die Schutzgruppe abspaltenden Reagenz zur Reaktion bringt. Die Schutzgruppenabspaltung erfolgt nach allgemein üblichen Methoden (siehe z. B.: T. W. Green, P. G. M. Wuts, "Protective Groups in Organic Synthesis", 2nd ed., John Wiley and Sons Inc. 1991) durch saure Reagentien wie z.B. Bromwasserstoff in Eisessig oder Trifluoressigsäure oder etherische HCl-Lösung oder hydrogenolytisch oder durch palladium- oder rhodiumkatalysierte Spaltung.

Die Verbindungen der allgemeinen Formel IV werden hergestellt, indem man eine Verbindung der allgemeinen Formel V 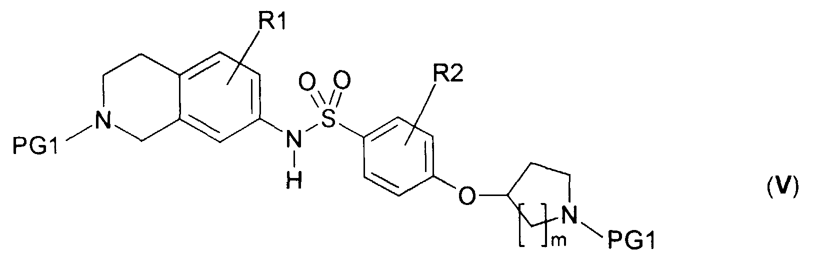 in der in der R¹, R², PG¹ und m die oben angegebenen Bedeutungen besitzen, mit Verbindungen des Typs R³-Y, bei dem R³ die oben angegebene Bedeutung besitzt und Y Halogen, Tosylat, Mesylat oder Triflat bedeutet, in einem inerten Lösungsmittel wie Dioxan, Tetrahydrofuran, N,N-Dimethylformamid, N-Methyl-pyrrolidon oder Toluol in Gegenwart einer Base wie beispielsweise Natriumcarbonat, Kaliumcarbonat, 1,5-Diazabicyclo[5.4.0]undec-5-en oder Ethyldiisopropylamin bei Temperaturen zwischen 0°C und dem Siedepunkt des Lösungsmittels, vorzugsweise zwischen Raumtemperatur und 80°C, umsetzt. Verbindungen des Typs R³-Y sind entweder käuflich oder literaturbekannt oder können aus käuflichen oder literaturbekannten Vorstufen nach Standardmethoden hergestellt werden.

Die Verbindungen der allgemeinen Formel V werden hergestellt, indem man eine Verbindung der allgemeinen Formel VI 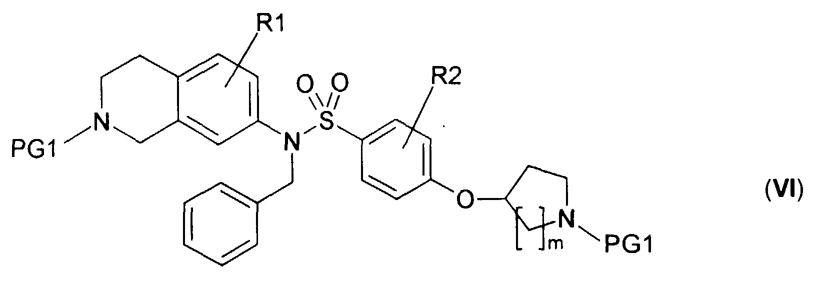 in der in der R¹, R², PG¹ und m die oben angegebenen Bedeutungen besitzen, einer katalytischen Hydrierung in inerten Lösungsmitteln wie z. B. Methanol, Ethanol, Tetrahydrofuran oder Dioxan in Gegenwart eines Katalysators, vorzugsweise Palladium auf Kohle, unterzieht. Die Benzylgruppe wird dabei durch ein Wasserstoffatom ersetzt. Die Entfernung der Benzylgruppe gelingt auch durch Umsetzung mit einer starken Säure wie Trifluoressigsäure in Gegenwart von Mesitylen, Anisol oder Thioanisol bei Temperaturen zwischen 0 und 50°C, vorzugsweise bei Raumtemperatur, oder durch Behandlung mit Lewissäuren wie BF₃-Etherat in einem inerten Lösungsmittel wie Toluol, Acetonitril, Diethylether oder Tetrahydrofuran bei Temperaturen zwischen 0°C und dem Siedepunkt des Lösungsmittels, vorzugsweise zwischen Raumtemperatur und dem Siedepunkt des Lösungsmittels.

Die Verbindungen der allgemeinen Formel VI stellt man her, in dem man eine Verbindung der allgemeinen Formel VII, 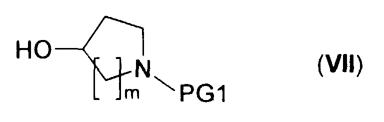 in der PG¹ und m die oben angegebenen Bedeutungen haben, mit einer Verbindung der allgemeinen Formel VIII, 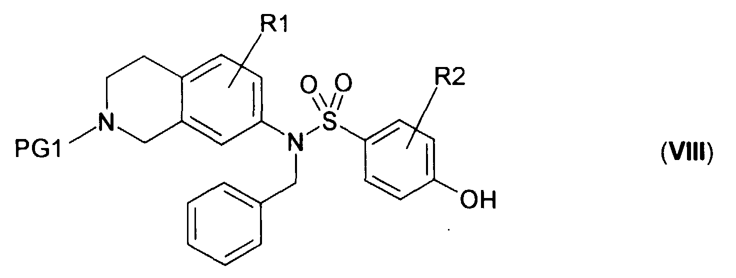 in der R¹, R² und PG¹ die oben angegebenen Bedeutungen haben, in einem inerten Lösungsmittel wie Dioxan, Tetrahydrofuran oder Toluol in Gegenwart von Diethylazodicarboxylat und Triphenylphosphin, Trimethyl- oder Triethylphosphit bei Temperaturen zwischen 0 und 50°C, vorzugsweise bei Raumtemperatur kondensiert.

Verbindungen der allgemeinen Formel VII, in der m und PG¹ die oben angegebenen Bedeutungen haben, sind entweder kauflich oder literaturbekannt oder können nach literaturbekannten Verfahren hergestellt werden (siehe z. B.: K. L. Bhat, D. M. Flanagan, M. M. Jouillé, *Synth. Commun.* **1985**, *15*, 587-598: P. G. Houghton, G. R. Humphrey, D. J. Kennedy, D. C. Roberts, S. H. Wright, *J. Chem. Soc. Perkin Trans. 1* **1993**, *13*, 1421-1424; T. W. Green, P. G. M. Wuts, "Protective Groups in Organic Synthesis", 2nd ed., John Wiley and Sons Inc. 1991).

Die Verbindungen der allgemeinen Formel VIII stellt man her, in dem man eine Verbindung der allgemeinen Formel IX, 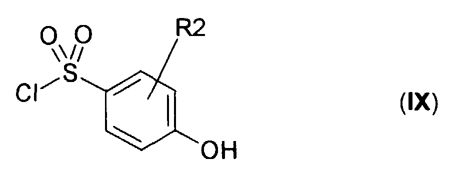 in der R² die oben angegebene Bedeutung hat, mit einer Verbindung der allgemeinen Formel X, 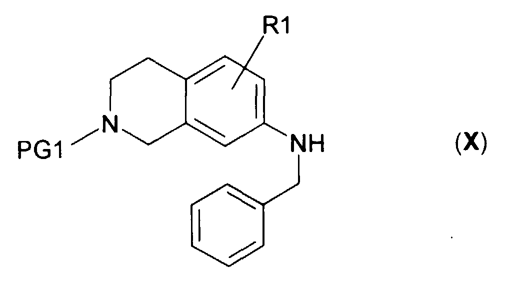 in der R¹ und PG¹ die oben angegebenen Bedeutungen haben, in einem inerten Lösungsmittel wie N,N-Dimethylformamid, N-Methylpyrrolidon, Dioxan, Tetrahydrofuran, Dichlormethan oder Toluol in Gegenwart einer Base wie beispielsweise Natriumcarbonat, Kaliumcarbonat, 1,5-Diazabicyclo[5.4.0]undec-5-en, Triethylamin, N-Methylmorpholin oder Ethyldiisopropylamin bei Temperaturen zwischen -20°C und dem Siedepunkt des Lösungsmittels, vorzugsweise zwischen 0°C und 80°C, umsetzt.

Verbindungen des Typs IX sind entweder käuflich oder literaturbekannt oder können aus käuflichen oder literaturbekannten Vorstufen nach Standardmethoden hergestellt werden (siehe z. B.: M. Sato, Y. Kawashima, J. Goto, Y. Yamane, Y. Chiba et. al., *Eur. J. Med. Chem. Chim. Ther*. **1995**, *30*, 403-414; W. Loewe. T. Braden, *Arch. Pharm*. *(Weinheim Ger.)* **1995**, *328*, 283-286; R. Cremlin, F. Swinbourne, J. Atherall, L. Courtney, T. Cronje et.al. *Phosphorus Sulfur* **1980**, *9*, 155-164; R. W. Campbell. H. W Hill, *J. Org. Chem.* **1973**, *38*, 1047, Stewart *J. Chem. Soc.* **1922**, *121*, 2559).

Die Verbindungen der allgemeinen Formel X stellt man her, in dem man eine Verbindung der allgemeinen Formel XI, 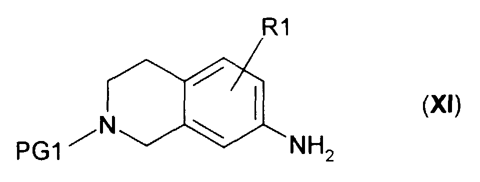 in der R¹ und PG¹ die oben angegebenen Bedeutungen haben, in einem inerten Lösungsmittel wie Dichlorethan, Tetrahydrofuran, Dioxan, Ethanol, Methanol oder Diglyme, in Gegenwart eines Reduktionsmittels wie beispielsweise Natriumborhydrid, Natriumcyanoborhydrid oder Natriumtriacetoxyborhydrid, bei Temperaturen zwischen - 20°C und dem Siedepunkt des Lösungsmittels, vorzugsweise zwischen 0°C und 60°C, umsetzt.

Die Verbindungen der allgemeinen Formel XI stellt man her, in dem man eine Verbindung der allgemeinen Formel XII, 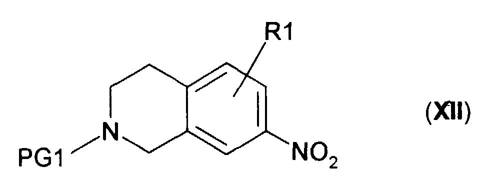 in der R¹ und PG¹ die oben angegebenen Bedeutungen haben, in einem inerten Lösungsmittel wie Methanol, Ethanol, Tetrahydrofuran oder Dioxan in Gegenwart eines Katalysators, beispielsweise Palladium auf Kohle, Tris-triphenylphosphin-rhodiumchlorid oder Raney-Nickel hydriert. Gegebenenfalls kann die Reduktion auch mit anderen Reduktionsmitteln als Wasserstoff durchgeführt werden, z. B mit Lithiumaluminiumhydrid, Natriumborhydrid / Kobaltdichlorid, Natriumborhydrid / Nickeldichlorid, Triethylsilan / Tris-triphenylphosphin-rhodiumchlorid oder mit unedlen Metallen wie Eisen oder Zinn in Gegenwart von Säure.

Die Verbindungen der allgemeinen Formel XII stellt man her, in dem man eine Verbindung der allgemeinen Formel XIII, 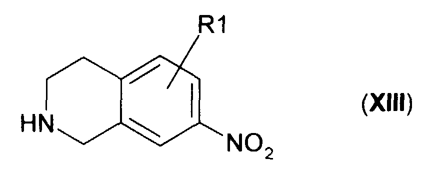 in der R¹ die oben angegebene Bedeutung hat, in einem inerten Lösungsmittel wie Methanol, Ethanol, Tetrahydrofuran, Dioxan, Dimethylformamid oder Wasser bei Temperaturen zwischen 0°C und Siedepunkt des Lösungsmittels, bevorzugt zwischen Raumtemperatur und 50°C, mit den entsprechenden, die Schutzgruppe einführenden, käuflichen Reagentien wie z. B. Di-tert.-butyldicarbonat oder Chlorameisensäureestern zur Reaktion bringt. Die Einführung der entsprechenden Schutzgruppe erfolgt nach den allgemein bekannten Methoden der Schutzgruppenchemie (siehe z. B.: T. W Green. P G M. Wuts, "Protective Groups in Organic Synthesis", 2nd ed., John Wiley and Sons Inc. 1991), wobei gegebenenfalls die Gegenwart von Basen wie Alkali- oder Erdalkalihydroxiden, Natriumcarbonat, Kaliumcarbonat, N-Methylmorpholin, Diisopropylethylamin, Triethylamin oder 1,5-Diazabicyclo[5.4.0]undec-5-en erforderlich ist.

Verbindungen des Typs XIII sind entweder käuflich oder literaturbekannt oder können aus käuflichen oder literaturbekannten Vorstufen nach Standardmethoden hergestellt werden (siehe z. B.: J. F. Ajao, C. W. Bird, *J. Heterocycl. Chem.* **1985**, *22*, 329-331; E. Ochai, T Nakagomo *Chem. Pharm. Bull.* **1958**, *6*, 497; A. McCoubrey, D. W. Mathieson, *J. Chem. Soc* **1951**, 2851).

Verbindungen der allgemeinen Formel I können auch hergestellt werden, indem man beispielsweise eine Verbindung der allgemeinen Formel XIV 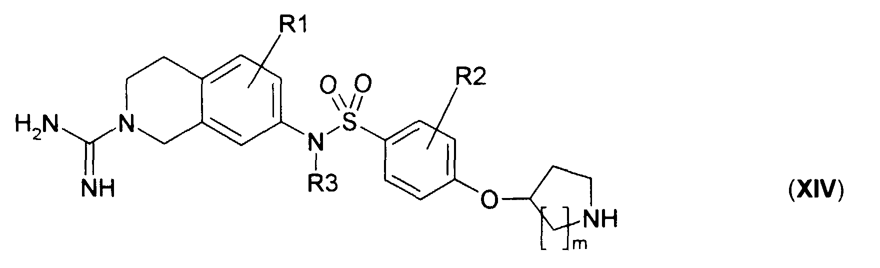 in der R¹, R², R³ und m die oben angegebenen Bedeutungen besitzen, mit aliphatischen oder aromatischen Imidsäureester-Hydrochloriden in einem inerten Lösungsmittel wie Tetrahydrofuran, Diethylether, Ethanol, Dimethylformamid oder Dioxan bei Temperaturen zwischen -20°C und Siedepunkt des Lösungsmittels, vorzugsweise zwischen 0°C und 40°C, in Gegenwart einer Hilfsbase wie Triethylamin. Diisopropylethylamin oder N-Methylmorpholin umsetzt.

Verbindungen der allgemeinen Formel XIV können hergestellt werden, indem man beispielsweise eine Verbindung der allgemeinen Formel XV 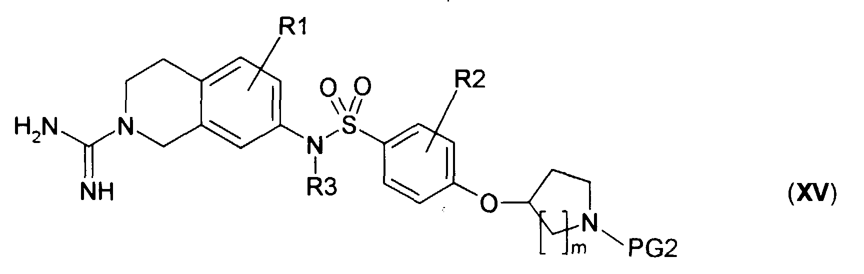 in der in der R¹, R², R³ und m die oben angegebenen Bedeutungen besitzen und PG² eine Schutzgruppe wie z. B. die Allyloxycarbonylgruppe bedeutet, mit einem die Schutzgruppe abspaltenden Reagenz zur Reaktion bringt. Die Schutzgruppenabspaltung erfolgt nach allgemein üblichen Methoden (siehe z. B.: T. W. Green, P. G. M. Wuts, "Protective Groups in Organic Synthesis", 2nd ed., John Wiley and Sons Inc. 1991) z. B. durch palladium- oder rhodiumkatalysierte Spaltung.

Verbindungen der allgemeinen Formel XV können hergestellt werden, indem man beispielsweise eine Verbindung der allgemeinen Formel XVI 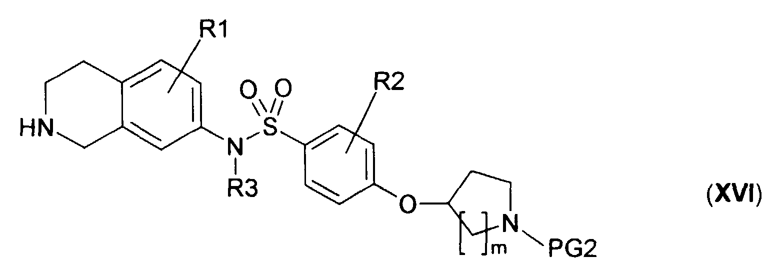 in der R¹, R², R³, m und PG² die oben angegebenen Bedeutungen besitzen, mit einem Guanylierungsreagenz wie beispielsweise 1H-Pyrazol-1-carboxamidin oder S-Methylisothioharnstoff in einem inerten Lösungsmittel wie z. B. Dimethylformamid, Dioxan. Dimethylsulfoxid oder Toluol bei Temperaturen zwischen 0°C und Siedepunkt des Lösungsmittels, vorzugsweise bei 0 bis 30°C in Gegenwart einer Hilfsbase wie z. B. Triethylamin, N-Methylmorpholin, Pyridin oder Ethyldiisopropylamin zur Reaktion bringt.

Die Verbindungen der allgemeinen Formel XVI werden hergestellt, indem man eine Verbindung der allgemeinen Formel XVII 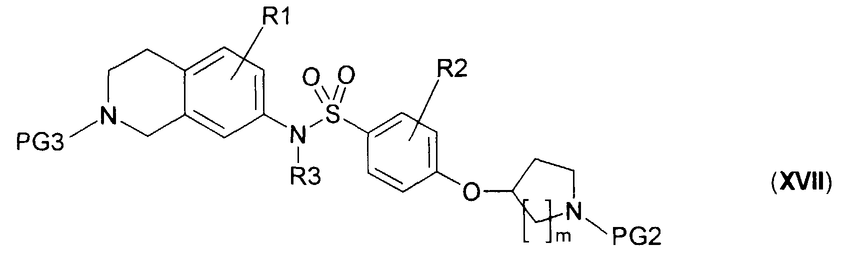 in der in der R¹, R², R³, m und PG² die oben angegebenen Bedeutungen besitzen und PG² ungleich PG³ ist, wobei PG³ eine Schutzgruppe wie z. B. die Benzyloxycarbonylgruppe, die t-Butyloxycarbonylgruppe oder die Allyloxycarbonylgruppe bedeutet, mit einem die selektiv die PG² Schutzgruppe abspaltenden Reagenz zur Reaktion bringt. Die Schutzgruppenabspaltung erfolgt nach allgemein üblichen Methoden (siehe z. B.: T. W. Green, P. G. M. Wuts, "Protective Groups in Organic Synthesis", 2nd ed., John Wiley and Sons Inc. 1991) z. B. durch saure Reagentien wie Bromwasserstoff in Eisessig oder Trifluoressigsäure oder etherische HCl-Lösung oder hydrogenolytisch.

Die Verbindungen der allgemeinen Formel XVII können analog zu den Verbindungen der allgemeinen Formel IV aus den entsprechenden Vorstufen hergestellt werden. Alternativ können die Verbindungen der allgemeinen Formel XVII auch hergestellt werden, indem man eine Verbindung der allgemeinen Formel XVIII, 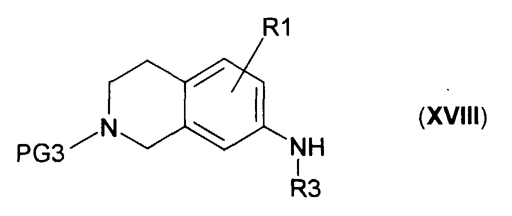 in der in der R¹, R³ und PG³ die oben angegebenen Bedeutungen besitzen, mit einer Verbindung der allgemeinen Formel XIX, 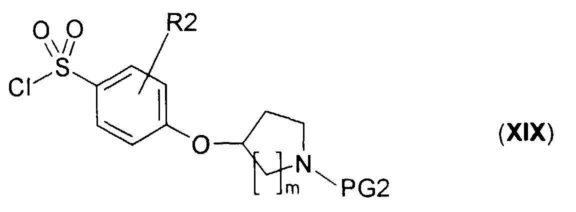 in der in der R², m, und PG² die oben angegebenen Bedeutungen besitzen, zur Reaktion bringt. Die Umsetzung geschieht in einem inerten Lösungsmittel wie N,N-Dimethylformamid, N-Methylpyrrolidon, Dioxan, Tetrahydrofuran, Dichlormethan oder Toluol in Gegenwart einer Base wie beispielsweise Natriumcarbonat, Kaliumcarbonat, 1,5-Diazabicyclo[5.4.0]undec-5-en, Triethylamin, N-Methylmorpholin oder Ethyldiisopropylamin bei Temperaturen zwischen -20°C und dem Siedepunkt des Lösungsmittels, vorzugsweise zwischen 0°C und 80°C,

Die Verbindungen der allgemeinen Formel XVIII können aus Verbindungen der allgemeinen Formel XX, 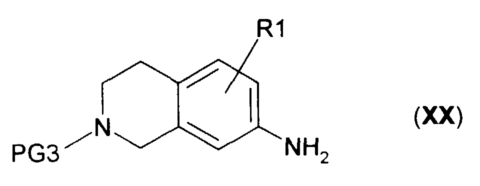 in der in der R¹ und PG³ die oben angegebenen Bedeutungen besitzen, durch Alkylierung mit Verbindungen des Typs R³-Y, bei dem R³ die oben angegebene Bedeutung besitzt und Y Halogen, Tosylat, Mesylat oder Triflat bedeutet, in einem inerten Lösungsmittel wie Dioxan, Tetrahydrofuran, N,N-Dimethylformamid, N-Methyl-pyrrolidon oder Toluol in Gegenwart einer Base wie beispielsweise Natriumcarbonat, Kaliumcarbonat, 1,5-Diazabicyclo[5.4.0]undec-5-en oder Ethyldiisopropylamin bei Temperaturen zwischen 0°C und dem Siedepunkt des Lösungsmittels, vorzugsweise zwischen Raumtemperatur und 80°C hergestellt werden. Verbindungen des Typs R³-Y sind entweder käuflich oder literaturbekannt oder können aus käuflichen oder literaturbekannten Vorstufen nach Standardmethoden hergestellt werden. Alternativ können die Verbindungen der allgemeinen Formel XVIII auch, ausgehend von Verbindungen der allgemeinen Formel XX, durch reduktive Aminierung des entsprechenden Aldehyds gewogen werden. Diese Umsetzungen erfolgen in einem inerten Lösungsmittel wie Dichlorethan, Tetrahydrofuran, Dioxan, Ethanol, Methanol oder Diglyme, in Gegenwart eines Reduktionsmittels wie beispielsweise Natriumborhydrid, Natriumcyanoborhydrid oder Natriumtriacetoxyborhydrid, bei Temperaturen zwischen -20°C und dem Siedepunkt des Lösungsmittels, vorzugsweise zwischen 0°C und 60°C. Die entsprechenden Aldehyde sind entweder käuflich oder literaturbekannt oder können aus käuflichen oder literaturbekannten Vorstufen nach Standardmethoden hergestellt werden. Die Verbindungen der allgemeinen Formel XX können analog zu den Verbindungen der allgemeinen Formel XI aus den entsprechenden Vorstufen hergestellt werden.

Die Verbindungen der allgemeinen Formel XIX können hergestellt werden, indem man eine Verbindung der allgemeinen Formel XXI, 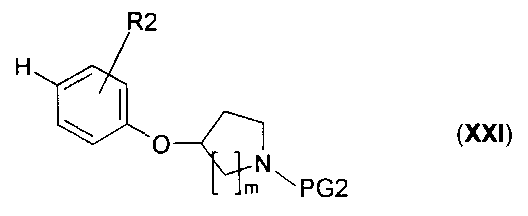 in der in der R², m und PG² die oben angegebenen Bedeutungen besitzen, sulfochloriert. Dies geschieht in einem inerten Lösungsmittel wie Dichlormethan, Chloroform oder Tetrachlorkohlenstoff unter Verwendung eines Sulfochlorierungsreagenzes wie Chlorsulfonsäure, gegebenenfalls in Gegenwart von Thionylchlorid, Sulfurylchlorid oder Phosphorylchlorid bei Temperaturen zwischen -20°C und dem Siedepunkt des Lösungsmittels, vorzugsweise zwischen 0°C und 40°C.

Die Verbindungen der allgemeinen Formel XXI stellt man her, in dem man eine Verbindung der allgemeinen Formel XXII, 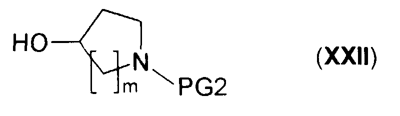 in der PG² und m die oben angegebenen Bedeutungen haben, mit einer Verbindung der allgemeinen Formel XXIII, 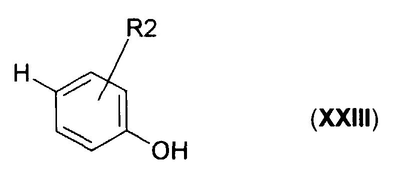 in der R² die oben angegebene Bedeutung hat, in einem inerten Lösungsmittel wie Dioxan, Tetrahydrofuran oder Toluol in Gegenwart von Diethylazodicarboxylat und Triphenylphosphin, Trimethyl- oder Triethylphosphit bei Temperaturen zwischen 0 und 50°C, vorzugsweise bei Raumtemperatur kondensiert.

Verbindungen der allgemeinen Formel XXII, in der m und PG² die oben angegebenen Bedeutungen haben, sind entweder käuflich oder literaturbekannt oder können nach literaturbekannten Verfahren hergestellt werden (siehe z. B.: K. L. Bhat, D. M. Flanagan, M. M. Jouillé, *Synth. Commun.* **1985**, *15*, 587-598; P. G. Houghton, G. R. Humphrey, D. J. Kennedy, D. C. Roberts, S. H. Wright, *J. Chem. Soc. Perkin Trans. 1* **1993,** *13*, 1421-1424; T. W. Green, P. G. M. Wuts, "Protective Groups in Organic Synthesis", 2nd ed., John Wiley and Sons Inc. 1991).

Verbindungen der allgemeinen Formel XXIII, in der R² die oben angegebene Bedeutung hat, sind entweder käuflich oder literaturbekannt oder können aus käuflichen oder literaturbekannten Vorstufen nach Standardmethoden hergestellt werden.

Gewisse Verbindungen der allgemeinen Formel I lassen sich nachträglich in andere Verbindungen der allgemeinen Formel I umwandeln.

Dies betrifft Verbindungen der allgemeinen Formel I, in der R¹, R², R³, R⁴, n, und m die oben angegebenen Bedeutungen haben und einer oder mehrere der Reste R¹, R², R³ eine Methoxygruppe bedeutet oder beinhaltet. Durch Behandlung mit gängigen, die Methylgruppe abspaltenden Reagenzien (siehe z. B.: T. W. Green, P. G. M. Wuts, "Protective Groups in Organic Synthesis", 2nd ed., John Wiley and Sons Inc. 1991) wie z. B. Trimethylsilyliodid oder Bortribromid in einem inerten Lösungsmittel wie Dichlormethan, Dichlorethan, Chloroform, Tetrachlorkohlenstoff Tetrahydrofuran, Dioxan, Aceton, Acetonitril bei Temperaturen zwischen 0°C und dem Siedepunkt des Lösungsmittels, vorzugsweise zwischen 0°C und 60°C, können diese Verbindungen in die entsprechenden Verbindungen der allgemeinen Formel I mit freier Hydroxygruppe umgewandelt werden.

Dies betrifft auch Verbindungen der allgemeinen Formel I, in der R¹, R², R³, R⁴, n, und m die oben angegebenen Bedeutungen haben und einer oder mehrere der Reste R¹, R² eine Ethoxy- oder Methoxycarbonylgruppe bedeutet oder beinhaltet. Durch saure oder alkalische Hydrolyse in einem inerten Lösungsmittel wie Tetrahydrofuran, Dioxan, Aceton, Ethanol, Methanol oder Wasser bei Temperaturen zwischen 0°C und dem Siedepunkt des Lösungsmittels, vorzugsweise zwischen Raumtemperatur und 60°C, können diese Verbindungen in die entsprechenden Verbindungen der allgemeinen Formel I mit freier Carboxylgruppe umgewandelt werden.

Dies betrifft auch Verbindungen der allgemeinen Formel I, in der R¹, R², R³, R⁴, n, und m die oben angegebenen Bedeutungen haben und einer oder mehrere der Reste R¹, R² eine Benzyloxygruppe bedeutet. Durch katalytische Hydrierung in inerten Lösungsmitteln wie z. B. Methanol, Ethanol, Tetrahydrofuran oder Dioxan in Gegenwart eines Katalysators, vorzugsweise Palladium auf Kohle, wird die Benzylgruppe dabei durch ein Wasserstoffatom ersetzt (siehe z. B.: T. W. Green, P. G. M. Wuts, "Protective Groups in Organic Synthesis", 2nd ed., John Wiley and Sons Inc. 1991). Die Entfernung der Benzylgruppe gelingt auch durch Umsetzung mit einer starken Säure wie Trifluoressigsäure in Gegenwart von Mesitylen, Anisol oder Thioanisol bei Temperaturen zwischen 0 und 50°C, vorzugsweise bei Raumtemperatur, oder durch Behandlung mit Lewissäuren wie Bortrifluorid-Etherat in einem inerten Lösungsmittel wie Toluol, Acetonitril, Diethylether oder Tetrahydrofuran bei Temperaturen zwischen 0°C und dem Siedepunkt des Lösungsmittels, vorzugsweise zwischen Raumtemperatur und dem Siedepunkt des Lösungsmittels.

Dies betrifft auch Verbindungen der allgemeinen Formel I, in der R¹, R², R³, R⁴, n, und m die oben angegebenen Bedeutungen haben und einer oder mehrere der Reste R¹, R² eine Allyloxygruppe bedeutet. Durch übergangsmetallkatalysierte Spaltung, beispielsweise in Gegenwart eines Rhodiumkatalysators wie Tris-triphenylphosphinrhodiumchlorid oder eines Palladiumkatalysators wie Tetrakis-triphenylphosphinpalladium in einem inerten Lösungsmittel wie Tetrahydrofuran oder Dioxan, gegebenenfalls in Anwesenheit eines Nucleophils wie z. B. Malonsäurediethylester, Tributylzinnhydrid, 5,5-Dimethyl-cyclohexan-1,3-dion oder Piperidin bei Temperaturen zwischen 0°C und 50°C, vorzugsweise bei Raumtemperatur, wird die Allylgruppe dabei durch ein Wasserstoffatom ersetzt (siehe z. B.: T. W. Green, P. G. M. Wuts, "Protective Groups in Organic Synthesis", 2nd ed., John Wiley and Sons Inc. 1991).

Zu reinen Enantiomeren der Verbindungen der Formel I kommt man entweder durch Racematspaltung (über Salzbildung mit optisch aktiven Säuren oder Basen), oder indem man in die Synthese optisch aktive Ausgangsstoffe einsetzt oder indem man enzymatisch hydrolysiert.

Bevorzugt im Sinne der Erfindung sind außer den in den Beispielen genannten Verbindungen die folgenden:
1. 7-{4-[1-(1-Imino-ethyl)-piperidin-4-yloxy]-benzolsulfonylamino}-3,4-dihydro-1H-isochinolin-2-carboxamidin
2. 7-{4-[1-(1-Iminopropyl)-piperidin-4-yloxy]-benzolsulfonylamino}-3,4-dihydro-1H-isochinolin-2-carboxamidin
3. 7-{4-[1-(Cyclopropyl-imino-methyl)-piperidin-4-yloxy]-benzolsulfonylamino}-3,4-dihydro-1H-isochinolin-2-carboxamidin
4. 7-(4-{1-[Imino-(4-methoxy-phenyl)-methyl]-piperidin-4-yloxy}-benzolsulfonylamino)-3,4-dihydro-1H-isochinolin-2-carboxamidin
5. 7-{4-[1-(Imino-thiophen-2-yl-methyl)-piperidin-4-yloxy]-benzolsulfonylamino}-3,4-dihydro-1H-isochinolin-2-carboxamidin
6. 7-{[4-(1-Carbamimidoyl-piperidin-4-yloxy)-benzolsulfonyl]-methyl-amino}-3,4-dihydro-1H-isochinolin-2-carboxamidin
7. 7-({4-[1-(1-Imino-ethyl)-piperidin-4-yloxy]-benzolsulfonyl}-methyl-amino)-3,4-dihydro-1H-isochinolin-2-carboxamidin
8. 7-({4-[1-(1-Imino-propyl)-piperidin-4-yloxy]-benzolsulfonyl}-methyl-amino)-3,4-dihydro-1H-isochinolin-2-carboxamidin
9. 7-({4-[1-(Cyclopropyl-imino-methyl)-piperidin-4-yloxy]-benzolsulfonyl}-methyl-amino)-3,4-dihydro-1H-isochinolin-2-carboxamidin
10. 7-[(4-{1-[Imino-(4-methoxy-phenyl)-methyl]-piperidin-4-yloxy}-benzolsulfonyl)-methyl-amino]-3,4-dihydro-1H-isochinolin-2-carboxamidin
11. 7-({4-[1-(Imino-thiophen-2-yl-methyl)-piperidin-4-yloxy]-benzolsulfonyl}-methyl-amino)-3,4-dihydro-1H-isochinolin-2-carboxamidin
12. 7-{[4-(1-Carbamimidoyl-piperidin-4-yloxy)-benzolsulfonyl]-ethyl-amino}-3,4-dihydro-1H-isochinolin-2-carboxamidin
13. 7-(Ethyl-{4-[1-(1-imino-ethyl)-piperidin-4-yloxy]-benzolsulfonyl}-amino)-3,4-dihydro-1H-isochinolin-2-carboxamidin
14. 7-(Ethyl-{4-[1-(1-imino-propyl)-piperidin-4-yloxy]-benzolsulfonyl}-amino)-3,4-dihydro-1H-isochinolin-2-carboxamidin
15. 7-({4-[1-(Cyclopropyl-imino-methyl)-piperidin-4-yloxy]-benzolsulfonyl}-ethyl-amino)-3,4-dihydro-1H-isochinolin-2-carboxamidin
16. 7-[Ethyl-(4-{1-[imino-(4-methoxy-phenyl)-methyl]-piperidin-4-yloxy}-benzolsulfonyl)-amino]-3,4-dihydro-1H-isochinolin-2-carboxamidin
17. 7-(Ethyl-{4-[1-(imino-thiophen-2-yl-methyl)-piperidin-4-yloxy]-benzolsulfonyl}-amino)-3,4-dihydro-1H-isochinolin-2-carboxamidin
18. 7-{[4-(1-Carbamimidoyl-piperidin-4-yloxy)-benzolsulfonyl]-cyclopropyl-amino}-3,4-dihydro-1H-isochinolin-2-carboxamidin
19. 7-(Cyclopropyl-{4[1-(1-imino-ethyl)-piperidin-4-yloxy]-benzolsulfonyl}-amino)-3,4-dihydro-1H-isochinolin-2-carboxamidin
20. 7-(Cyclopropyl-{4-[1-(1-imino-propyl)-piperidin-4-yloxy]-benzolsulfonyl}-amino)-3,4-dihydro-1H-isochinolin-2-carboxamidin
21. 7-(Cyclopropyl-{4-[1-(cyclopropyl-imino-methyl)-piperidin-4-yloxy]-benzolsulfonyl}-amino)-3,4-dihydro-1H-isochinolin-2-carboxamidin
22. 7-[Cyclopropyl-(4-{1-[imino-(4-methoxy-phenyl)-methyl]-piperidin-4-yloxy}-benzolsulfonyl)-amino]-3,4-dihydro-1H-isochinolin-2-carboxamidin
23. 7-(Cyclopropyl-{4-[1-(imino-thiophen-2-yl-methyl)-piperidin-4-yloxy]-benzolsulfonyl}-amino)-3,4-dihydro-1H-isochinolin-2-carboxamidin
24. 7-{Allyl-[4-(1-carbamimidoyl-piperidin-4-yloxy)-benzolsulfonyl]-amino}-3,4-dihydro-1H-isochinolin-2-carboxamidin
25. 7-(Allyl-{4-[1-(1-imino-ethyl)-piperidin-4-yloxy]-benzolsulfonyl}-amino)-3,4-dihydro-1H-isochinolin-2-carboxamidin
26. 7-(Allyl-{4-[1-(1-imino-propyl)-piperidin-4-yloxy]-benzolsulfonyl}-amino)-3,4-dihydro-1H-isochinolin-2-carboxamidin
27. 7-(Allyl-{4-[1-(cyclopropyl-imino-methyl)-piperidin-4-yloxy]-benzolsulfonyl}-amino)-3,4-dihydro-1H-isochinolin-2-carboxamidin
28. 7-[Allyl-(4-{1-[imino-(4-methoxy-phenyl)-methyl]-piperidin-4-yloxy}-benzolsulfonyl)-amino]-3,4-dihydro-1H-isochinolin-2-carboxamidin
29. 7-(Allyl-{4-[1-(imino-thiophen-2-yl-methyl)-piperidin-4-yloxy]-benzolsulfonyl}-amino)-3,4-dihydro-1H-isochinolin-2-carboxamidin
30. 7-{Benzyl-[4-(1-carbamimidoyl-piperidin4-yloxy)-benzolsulfonyl]-amino}-3,4-dihydro-1H-isochinolin-2-carboxamidin
31. 7-(Benzyl-{4-[1-(1-imino-ethyl)-piperidin-4-yloxy]-benzolsulfonyl}-amino)-3,4-dihydro-1H-isochinolin-2-carboxamidin
32. 7-(Benzyl-{4-[1-(1-imino-propyl)-piperidin-4-yloxy]-benzolsulfonyl}-amino)-3,4-dihydro-1H-isochinolin-2-carboxamidin
33. 7-(Benzyl-{4-[1-(cyclopropyl-imino-methyl)-piperidin-4-yloxy]-benzolsulfonyl}-amino)-3,4-dihydro-1H-isochinolin-2-carboxamidin
34. 7-[Benzyl-(4-{1-[imino-(4-methoxy-phenyl)-methyl]-piperidin-4-yloxy)-benzolsulfonyl}-amino]-3,4-dihydro-1H-isochinolin-2-carboxamidin
35. 7-(Benzyl-{4-[1-(imino-thiophen-2-yl-methyl)-piperidin-4-yloxy]-benzolsulfonyl}-amino)-3,4-dihydro-1H-isochinolin-2-carboxamidin
36. 7-[[4-(1-Carbamimidoyl-piperidin-4-yloxy)-benzolsulfonyl]-(2-hydroxy-ethyl)-amino]-3,4-dihydro-1H-isochinolin-2-carboxamidin
37. 7-((2-Hydroxy-ethyl)-{4-[1-(1-imino-ethyl)-piperidin-4-yloxy]-benzolsulfonyl}-amino)-3,4-dihydro-1H-isochinolin-2-carboxamidin
38. 7-((2-Hydroxy-ethyl)-{4-[1-(1-imino-propyl)-piperidin-4-yloxy]-benzolsulfonyl}-amino)-3,4-dihydro-1H-isochinolin-2-carboxamidin
39. 7-[{4-[1-(Cyclopropyl-imino-methyl)-piperidin-4-yloxy]-benzolsulfonyl}-(2-hydroxyethyl)-amino]-3,4-dihydro-1H-isochinolin-2-carboxamidin
40. 7-[(2-Hydroxy-ethyl)-(4-{1-[imino-(4-methoxy-phenyl)-methyl]-piperidin-4-yloxy}-benzolsulfonyl)-amino]-3,4-dihydro-1H-isochinolin-2-carboxamidin
41. 7-((2-Hydroxy-ethyl)-{4-[1-(imino-thiophen-2-yl-methyl)-piperidin-4-yloxy]-benzolsulfonyl}-amino)-3,4-dihydro-1H-isochinolin-2-carboxamidin
42. [[4-(1-Carbamimidoyl-piperidin-4-yloxy)-benzolsulfonyl]-(2-carbamimidoyl-1,2,3,4-tetrahydro-isoquinolin-7-yl)-amino]-essigsäure
43. ((2-Carbamimidoyl-1,2,3,4-tetrahydro-isoquinolin-7yl)-{4-[1-(1-imino-ethyl)-piperidin-4-yloxy]-benzolsulfonyl}-amino)-essigsäure
44. ((2-Carbamimidoyl-1,2,3,4-tetrahydro-isoquinolin-7yl)-{4-[1-(1-imino-propyl)-piperidin-4-yloxy]-benzolsulfonyl}-amino)-essigsäure
45. ((2-Carbamimidoyl-1,2,3,4-tetrahydro-isoquinolin-7yl)-{4-[1-(cyclopropyl-imino-methyl)-piperidin-4-yloxy]-benzolsulfonyl}-amino)-essigsäure
46. [(2-Carbamimidoyl-1,2,3,4-tetrahydro-isoquinolin-7-yl)-(4-{1-[imino-(4-methoxy-phenyl)-methyl]-piperidin-4-yloxy}-benzolsulfonyl)-amino]-essigsäure
47. ((2-Carbamimidoyl-1,2,3,4-tetrahydro-isoquinolin-7-yl)-{4-[1-(imino-thiophen-2-yl-methyl)-piperidin-4-yloxy]-benzolsulfonyl}-amino)-essigsäure
48. 4-[[4-(1-Carbamimidoyl-piperidin-4-yloxy)-benzolsulfonyl]-(2-carbamimidoyl-1,2,3,4-tetrahydro-isoquinolin-7-yl)-amino]-buttersäure
49. 4-((2-Carbamimidoyl-1,2,3,4-tetrahydro-isoquinolin-7-yl)-{4-[1-(1-imino-ethyl)-piperidin-4-yloxy]-benzolsulfonyl}-amino)-buttersäure
50. 4-((2-Carbamimidoyl-1,2,3,4-tetrahydro-isoquinolin-7-yl)-{4-[1-(1-imino-propyl)-piperidin-4-yloxy]-benzolsulfonyl}-amino)-buttersäure
51. 4-((2-Carbamimidoyl-1,2,3,4-tetrahydro-isoquinolin-7-yl)-{4-(1-(cyclopropyl-imino-methyl)-piperidin-4-yloxy]-benzolsulfonyl}-amino)-buttersäure
52. 4-[(2-Carbamimidoyl-1,2,3,4-tetrahydro-isoquinolin-7-yl)-(4-{1-[imino-(4-methoxy-phenyl)-methyl]-piperidin-4-yloxy}-benzolsulfonyl)-amino]-buttersäure
53. 4-((2-Carbamimidoyl-1,2,3,4-tetrahydro-isoquinolin-7-yl)-{4-[1-(imino-thiophen-2-yl-methyl)-piperidin-4-yloxy]-benzolsulfonyl}-amino)-buttersäure
54. [[4-(1-Carbamimidoyl-piperidin-4-yloxy)-benzolsulfonyl]-(2-carbamimidoyl-1,2,3,4-tetrahydro-isoquinolin-7-yl)-amino]-essigsäureethylester
55. ((2-Carbamimidoyl-1,2,3,4-tetrahydro-isoquinolin-7-yl)-{4-[1-(1-imino-ethyl)-piperidin-4-yloxy]-benzolsulfonyl}-amino)-essigsäureethylester
56. ((2-Carbamimidoyl-1,2,3,4-tetrahydro-isoquinolin-7-yl)-{4-[1-(1-imino-propyl)-piperidin-4-yloxy]-benzolsulfonyl}-amino)-essigsäureethylester
57. ((2-Carbamimidoyl-1,2,3,4-tetrahydro-isoquinolin7-yl)-{4-[1-(cyclopropyl-imino-methyl)-piperidin-4-yloxy]-benzolsulfonyl}-amino)-essigsäureethylester
58. [(2-Carbamimidoyl-1,2,3,4-tetrahydro-isoquinolin-7-yl)-(4-{1-[imino-(4-methoxy-phenyl)-methyl]-piperidin-4-yloxy}-benzolsulfonyl)-amino]-essigsäureethylester
59. ((2-Carbamimidoyl-1,2,3,4-tetrahydro-isoquinolin-7-yl)-{4-[1-(imino-thiophen-2-yl-methyl)-piperidin-4-yloxy]-benzolsulfonyl}-amino)-essigsäureethylester
60. 7-{Acetyl-[4-(1-carbamimidoyl-piperidin-4-yloxy)-benzolsulfonyl]-amino}-3,4-dihydro-1H-isochinolin-2-carboxamidin
61. 7-(Acetyl-{4-[1-(1-imino-ethyl)-piperidin-4-yloxy]-benzolsulfonyl}-amino)-3,4-dihydro-1H-isochinolin-2-carboxamidin
62. 7-(Acetyl-{4-[1-(1-imino-propyl)-piperidin-4-yloxy]-benzolsulfonyl}-amino)-3,4-dihydro-1H-isochinolin-2-carboxamidin
63. 7-(Acetyl-{4-[1-(cyclopropyl-imino-methyl)-piperidin-4-yloxy]-benzolsulfonyl}-amino)-3,4-dihydro-1H-isochinolin-2-carboxamidin
64. 7-[Acetyl-(4-{1-[imino-(4-methoxy-phenyl)-methyl]-piperidin-4-yloxy}-benzolsulfonyl)-amino]-3,4-dihydro-1H-isochinolin-2-carboxamidin
65. 7-(Acetyl-{4-[1-(imino-thiophen-2-yl-methyl)-piperidin-4-yloxy]-benzolsulfonyl}-amino)-3,4-dihydro-1H-isochinolin-2-carboxamidin
66. 7-[[4-(1-Carbamimidoyl-piperidin-4-yloxy)-benzolsulfonyl]-(4-methoxy-benzoyl)-amino]-3,4-dihydro-1H-isochinolin-2-carboxamidin
67. 7-[{4-[1-(1-Imino-ethyl)-piperidin-4-yloxy]-benzolsulfonyl}-(4-methoxy-benzoyl)-amino]-3,4-dihydro-1H-isochinolin-2-carboxamidin
68. 7-[{4-[1-(1-Imino-propyl)-piperidin-4-yloxy]-benzolsulfonyl}-(4-methoxy-benzoyl)-amino]-3,4-dihydro-1H-isochinolin-2-carboxamidin
69. 7-[{4-[1-(Cyclopropyl-imino-methyl)-piperidin-4-yloxy]-benzolsulfonyl}-(4-methoxy-benzoyl)-amino]-3,4-dihydro-1H-isochinolin-2-carboxamidin
70. 7-[(4-{1-[Imino-(4-methoxy-phenyl)-methyl]-piperidin-4-yloxy}-benzolsulfonyl)-(4-methoxy-benzoyl)-amino]-3,4-dihydro-1H-isochinolin-2-carboxamidin
71. 7-[{4-[1-(Imino-thiophen-2-yl-methyl)-piperidin-4-yloxy]-benzolsulfonyl}-(4-methoxy-benzoyl)-amino]-3,4-dihydro-1H-isochinolin-2-carboxamidin
72. [[4-(1-Carbamimidoyl-piperidin-4-yloxy)-benzolsulfonyl]-(2-carbamimidoyl-1,2,3,4-tetrahydro-isoquinolin-7-yl)-amino]-sulfonylessigsäure
73. ((2-Carbamimidoyl-1,2,3,4-tetrahydro-isoquinolin-7-yl)-{4-[1-(1-imino-ethyl)-piperidin-4-yloxy]-benzolsulfonyl}-amino)-sulfonylessigsäure
74. ((2-Carbamimidoyl-1,2,3,4-tetrahydro-isoquinolin-7-yl)-{4-[1-(1-imino-propyl)-piperidin-4-yloxy]-benzolsulfonyl}-amino)-sulfonylessigsäure
75. ((2-Carbamimidoyl-1,2,3,4-tetrahydro-isoquinolin-7-yl)-{4-[1-(cyclopropyl-imino-methyl)-piperidin-4-yloxy]-benzolsulfonyl}-amino)-sulfonylessigsäure
76. [(2-Carbamimidoyl-1,2,3,4-tetrahydro-isoquinolin-7-yl)-(4-{1-[imino-(4-methoxy-phenyl)-methyl]-piperidin-4-yloxy}-benzolsulfonyl)-amino]-sulfonylessigsäure
77. ((2-Carbamimidoyl-1,2,3,4-tetrahydro-isoquinolin-7-yl)-{4-[1-(imino-thiophen-2-yl-methyl)-piperidin-4-yloxy]-benzolsulfonyl}-amino)-sulfonylessigsäure
78. [[4-(1-Carbamimidoyl-piperidin-4-yloxy)-benzolsulfonyl]-(2-carbamimidoyl-1,2,3,4-tetrahydro-isoquinolin-7-yl)-amino]-methylboronsäure
79. ((2-Carbamimidoyl-1,2,3,4-tetrahydro-isoquinolin-7-yl)-{4-[1-(1-imino-ethyl)-piperidin-4-yloxy]-benzolsulfonyl}-amino)-methylboronsäure
80. ((2-Carbamimidoyl-1,2,3,4-tetrahydro-isoquinolin-7yl)-{4-[1-(1-imino-propyl)-piperidin-4-yloxy]-benzolsulfonyl}-amino)-methylboronsäure
81. ((2-Carbamimidoyl- 1,2,3,4-tetrahydro-isoquinolin-7-yl)-{4-[1-(cyclopropyl-imino-methyl)-piperidin-4-yloxy]-benzolsulfonyl} -amino)-methylboronsäure
82. [(2-Carbamimidoyl-1,2,3,4-tetrahydro-isoquinolin-7-yl)-(4-{1-[imino-(4-methoxy-phenyl)-methyl]-piperidin-4-yloxy}-benzolsulfonyl)-amino]-methylboronsäure
83. ((2-Carbamimidoyl-1,2,3,4-tetrahydro-isoquinolin-7-yl)-{4-[1-(imino-thiophen-2-yl-methyl)-piperidin-4-yloxy]-benzolsulfonyl}-amino)-methylboronsäure
84. 7-[4-(1-Carbamimidoyl-pyrrolidin-3-(S)-yloxy)-benzolsulfonylamino]-3,4-dihydro-1H-isochinolin-2-carboxamidin
85. [[4-(1-Carbamimidoyl-pyrrolidin-3-(S)-yloxy)-benzolsulfonyl]-(2-carbamimidoyl-1,2,3,4-tetrahydro-isoquinolin-7-yl)-amino]-sulfonylessigsäure
86. ((2-Carbamimidoyl-1,2,3,4-tetrahydro-isoquinolin-7-yl)-{4-[1-(1-imino-ethyl)-pyrrolidin-3-(S)-yloxy]-benzolsulfonyl}-amino)-sulfonylessigsäure
87. [[4-(1-Carbamimidoyl-pyrrolidin-3-(S)-yloxy)-benzolsulfonyl]-(2-carbamimidoyl-1,2,3,4-tetrahydro-isoquinolin-7yl)-amino]-essigsäure
88. ((2-Carbamimidoyl-1,2,3,4-tetrahydroisoquinolin-7-yl)-{4-[1-(1-imino-ethyl)-pyrrolidin-3-(S)-yloxy]-benzolsulfonyl}-amino)-essigsäure

Das folgende Beispiel legt die Erfindung exemplarisch dar, ohne sie jedoch darauf einzuschränken.

### Beispiel 1:

### 7-[4-(1-Carbamimidoyl-piperidin-4-yloxy)-benzolsulfonylamino]-3,4-dihydro-1H-isochinolin-2-carboxamidin-dihydrochlorid

### 1. 7-Nitro-3,4-dihydro-1H-isochinolin-2-carbonsäure-tert-butylester

Zu einer Suspension von 8.6 g (0.040 mol) 7-Nitro-1,2,3,4-tetrahydroisochinolin-hydrochlorid (J. F. Ajao, C. W. Bird, *J. Heterocycl. Chem.* **1985**, *22*, 329-331) und 16.6 ml (0.170 mol) Triethylamin in 100 ml Methylenchlorid wird bei 5°C eine Lösung von 9.6 g (0.044 mol) Di-tert-butyl-dicarbonat in 100 ml Methylenchlorid getropft. Nach 24-stdg. Rühren bei Raumtemperatur wird die entstandene klare Lösung nacheinander jeweils 3 x mit je 50 ml Wasser, 1 N Essigsäure und gesättigter Natriumbicarbonat-Lösung ausgeschüttelt. Nach Trocknen und Einengen wird der Rückstand mit Isohexan verrieben, abgesaugt und getrocknet. Man erhält 10.9 g (0.039 mol; 98 %) der Titelverbindung mit Schmp. 137-139°C als bräunlichen Feststoff EI-MS: 278 (M⁺).

### 2. 7-Amino-3,4-dihydro-1H-isochinolin-2-carbonsäure-tert-butylester

8.3 g (0.030 mol) 7-Nitro-3,4-dihydro-1H-isochinolin-2-carbonsäure-tert-butylester werden in 100 ml Essigester gelöst und in Gegenwart von 1.0 g Palladium/Kohle (10%) unter Normaldruck 5 h bei Raumtemperatur hydriert. Nach Aufnahme von 2160 ml Wasserstoff wird vom Katalysator abfiltriert und das Lösungsmittel abgezogen. Man erhält als Rückstand 7.3 g (0. 029 mol; 98 %) der Titelverbindung als hellbraunen Feststoff mit Schmp. 75-77°C; EI-MS: 248 (M⁺).

### 3. 7-Benzylamino-3,4-dihydro-1H-isochinolin-2-carbonsäure-tert-butylester

Eine Lösung von 5.0 g (0.020 mol) 7-Amino-3,4-dihydro-1H-isochinolin-2-carbonsäure-tert-butylester in 50 ml Methanol wird mit 2.40 ml (0.022 mol) Benzaldehyd versetzt und 16 h bei Raumtemperatur gerührt. Das resultierende Reaktionsgemisch wird auf 5°C abgekühlt und portionsweise 0.76 g (0.021 mol) Natriumborhydrid zugegeben. Nach 24stdg. Rühren bei Raumtemperatur wird das Methanol abdestilliert und der feste Rückstand mit Wasser verrieben, abgesaugt und getrocknet. Man erhält 5.80 g (86%) der Titelverbindung als weissen Feststoff Schmp. 110°C; (+)-FAB-MS: 339 (MH⁺).

### 4. 7-[Benzyl-(4-hydroxy-benzolsulfonyl)-amino]-3,4-dihydro-1H-isochinolin-2-carbonsäure-tert-butylester

Eine Lösung von 9.1 g (0.027 mol) 7-Benzylamino-3,4-dihydro-1H-isochinolin-2-carbonsäure-tert-butylester in 150 ml abs. Pyridin wird bei 5°C portionsweise mit 6.24 g (0.030 mol) 4-Hydroxybenzolsulfonylchlorid (R. W. Campbell, H. W. Hill, *J. Org. Chem.* **1973**, *38*, 1047) versetzt und 16 h bei Raumtemperatur gerührt. Das Pydidin wird abdestilliert und der Rückstand in 150 ml Ethylacetat gelöst. Man extrahiert nacheinander jeweils 2 x mit je 50 ml Wasser, 1 N Essigsäure und gesättigter Natriumbicarbonat-Lösung mit Wasser verrieben, und trocknet die organische Phase über Natriumsulfat. Nach Einengen wird der Rückstand zur Reinigung an einer Kieselgelsäule chromatographiert (Laufmittel: Isohexan / Ethylacetat 8:2, 7:3, 6:4, 1:1). Nach Einengen der entsprechenden Säulenfraktionen erhält man 10.9 g (82%) der Titelverbindung als weissen, kristallinen Feststoff mit Schmp 172-175°C. EI-MS: 495 (M⁺).

### 5. 7-{Benzyl-[4-(1-tert-butoxycarbonyl-piperidin-4-yloxy)-benzolsulfonyl]-amino}-3,4-dihydro-1H-isochinolin-2-carbonsäure-tert-butylester

Eine Lösung von 9.2 g (0.019 mol) 7-[Benzyl-(4-hydroxy-benzolsulfonyl)-amino]-3,4-dihydro-1H-isochinolin-2-carbonsäure-tert-butylester, 4.1 g (0.021 mol) 4-Hydroxy-piperidin-1-carbonsäure-tert-butylester (analog K. L. Bhat, D. M Flanagan, M. M. Jouillé, *Synth. Commun.* **1985**, *15*, 587-598) und 5.4 g (0.021 mol) Triphenylphosphin in 150 ml Tetrahydrofuran wird bei 5°C mit 3.3 ml (0.021 mol) Azodicarbonsäurediethylester versetzt und 24 h bei Raumtemperatur gerührt. Nach Einengen wird der Rückstand zur Reinigung an einer Kieselgelsäule chromatographiert (Laufmittel: Isohexan/Ethylacetat 9:1, 8:2, 7:3). Nach Einengen der entsprechenden Säulenfraktionen erhält man 9.0 g (71%) der Titelverbindung als weissen Feststoff. Schmp. 142-144°C; (+)-FAB-MS: 678 (MH⁺).

### 6. 7-{[4-(1-tert-butoxycarbonyl-piperidin-4-yloxy)-benzolsulfonyl]-amino}-3,4-dihydro-1H-isochinolin-2-carbonsäure-tert-butylester

8.9 g 7-{Benzyl-[4-(1-tert-butoxycarbonyl-piperidin-4-yloxy)-benzolsulfonyl]-amino}-3,4-dihydro-1H-isochinolin-2-carbonsäure-tert-butylester (0.013mol) werden in 250 ml Essigester gelöst und in Gegenwart von 2.0 g Palladium/Kohle (10%) unter Normaldruck 10 d bei Raumtemperatur hydriert. Man filtriert vom Katalysator ab und engt ein. Der Rückstand wird zur Reinigung an einer Kieselgelsäule chromatographiert (Laufmittel: Isohexan/Ethylacetat 9:1, 8:2, 7:3). Nach Einengen der entsprechenden Säulenfraktionen erhält man 5.3 g (69%) der Titelverbindung als farbloses Öl. (+)-FAB-MS: 588 (MH⁺).

### 6. 4-(Piperidin-4-yloxy)-N-(1,2,3,4-tetrahydro-isochinolin-7-yl)-benzolsulfonamid-dihydrochlorid

Eine Lösung von 2.5 g (0.004 mol) 7-{[4-(1-tert-butoxycarbonyl-piperidin-4-yloxy)-benzolsulfonyl]-amino )-3,4-dihydro-1H-isochinolin-2-carbonsäure-tert-butylester in 50 ml Diethylether wird bei 5°C mit 50 ml etherischer HCl-Lösung versetzt und anschließend 5 h bei 5°C gerührt. Der ausgefallene weiße Feststoff wird abfiltriert und getrocknet: 1.9 g (0.0126 mmol; 70.1%); Schmp. 107-109°C: EI-MS: 387 (M⁺).

### 7. 7-[4-(1-Carbamimidoyl-piperidin-4-yloxy)-benzolsulfonylamino]-3,4-dihydro-1H-isochinolin-2-carboxamidin-dihydrochlorid

Eine Lösung von 0.9 g (0.002 mol) 4-(Piperidin-4-yloxy)-N-(1,2,3,4-tetrahydro-isochinolin-7-yl)-benzolsulfonamid-dihydrochlorid und 1.2 g (0.008 mol) 1H-Pyrazol-1-carboxamidin-hydrochlorid (Lit.: M. S. Bernatowicz, Y. Wu, G. R. Matsueda, *J. Org. Chem.* **1992**, *57*, 2497-2502) in 2 ml Dimethylformamid wird bei 5°C mit 4.2 ml Diisppropylethylamin versetzt. Nach 24stdg. Rühren bei Raumtemperatur wird 4 x mit je 25 ml Diethylether versetzt und abdekantiert. Der verbleibende Rückstand wird in 15 ml Wasser gelöst, mit 2 N HCl auf pH 3 eingestellt und mittels präparativer HPLC (RP-18-Säule, 15-25µm) chromatographiert (Laufmittel: H₂O, pH 3; H₂O/CH₃OH 6:4, pH 3). Nach Einengen der entsprechenden Säulenfraktionen und Trocknen i. Vak. (10⁻² Torr) erhält man 0.8 g (74 %) der Titelverbindung als weißen Feststoff mit Schmp. 150°C (Zers.); (+)-FAB-MS: 472(MH⁺).

### Beispiel 2:

### Pharmakologische Versuchsbeschreibung

### Plasmagewinnung

Neun Teile frisches Blut gesunder Spender werden mit einem Teil Natriumcitratlösung (0.11 Mol/l) gemischt und bei ca. 3000 U/min 10 Minuten bei Raumtemperatur zentrifügiert. Das Plasma wird abpipettiert und kann bei Raumtemperatur ca. 8 h aufbewahrt werden.

### Aktivierte partielle Thromboplastinzeit (APTT)

100 µl Citratplasma und 100 µl APTT-Reagenz (Diagnostica Stago / Boehringer Mannheim GmbH; enthält Lyophilisat Cephalin mit mikrokristallinem Kieselguraktivator) werden zusammen mit 10 µl Dimethylsulfoxid (DMSO) oder 10 µl einer Lösung der Wirksubstanz in DMSO in einem Kugelkoagulometer (KC10 der Fa. Amelung) 3 Minuten bei 37°C inkubiert. Mit Zugabe von 100 µl 0.025 M Calciumchlorid-Lösung wird eine Stoppuhr gestartet und der Zeitpunkt bis zum Eintritt der Gerinnung bestimmt. Die APTT beträgt bei den Kontrollmessungen ca. 28 -35 Sekunden und wird durch Wirksubstanzen verlängert. Sofern bei den Messungen nach 5 Minuten keine Gerinnung eintrat, wurde der Versuch gestoppt (>300).

In Tabelle 1 sind die gemessenen APTT-Zeiten in Sekunden als Differenz zur Kontrolle angegeben. Die Konzentrationen der Wirksubstanzen betrugen im Endvolumen 1000 µM (APTT 1000), 100 µM (APTT 100), 10 µM (APTT 10), 1 µM (APTT 1).

### Thrombinzeit

200 µl Citratplasma werden in einem Kugelkoagulometer (KC10 der Fa. Amelung) 2 Minuten bei 37°C inkubiert. Zu 190 µl vortemperiertem Thrombin-Reagenz (Boehringer Mannheim GmbH; enthält ca. 3 U/ml Pferdethrombin und 0.0125 M Ca⁺⁺ gibt man 10 µl Dimethylsulfoxid (DMSO) oder eine Lösung der Wirksubstanz in DMSO. Mit Zugabe dieser 200 µl Lösung zum Plasma wird eine Stoppuhr gestartet und der Zeitpunkt bis zum Eintritt der Gerinnung bestimmt. Die Thrombinzeit beträgt bei den Kontrollmessungen ca. 24 Sekunden und wird durch Wirksubstanzen verlängert. Sofern bei den Messungen nach 5 Minuten keine Gerinnung eintrat, wurde der Versuch gestoppt (>300).

In Tabelle 1 sind die gemessenen Thrombinzeiten in Sekunden als Differenz zur Kontrolle angegeben. Die Konzentrationen der Wirksubstanzen betrugen im Endvolumen 500 µM (TT 500).

### Inhibitionskonstanten

Die kinetischen Messungen wurden in 0.2 M Kochsalz und 0.5 % Polyethylenglycol 6000 enthaltendem 0.1 M Phosphatpuffer (Herstellung siehe unten) bei pH 7.5 und 25°C in Polystyrol-Halbmikroküvetten in einem Gesamtvolumen von 1 ml durchgeführt . Die Reaktionen wurden durch Zugabe von Enzym zu vorinkubierten Lösungen, die entweder Dimethylsulfoxid (Kontrolle) oder Lösungen der Testsubstanz in DMSO (Inhibitor-Stocklösungen: 10 mM in DMSO) enthielten, gestartet. Die Zunahme der Extinktion bei 405 nm als Folge der Freisetzung von 4-Nitroanilin aus dem Substrat wurde photometrisch über einen Zeitraum von 12 Minuten verfolgt. Im Abstand von 20 Sekunden wurden Meßwerte (Extinktion vs. Zeit) ermittelt und diese Daten per Computer gespeichert.

Zur Bestimmung der Inhibitionskonstanten Kᵢ wurde wie folgt vorgegangen: Die Geschwindigkeiten V₀ (Extinktionsänderung pro Sekunde; Messungen ohne Inhibitor) und Vᵢ (Extinktionsänderung pro Sekunde; Messungen mit Inhibitor) wurden durch lineare Regression bestimmt, wobei nur die Meßpunkte berücksichtigt wurden, bei der sich die Substratkonzentration um weniger als 15% verringerte. Aus einer Meßreihe (konstante Inhibitorkonzentration, variable Substratkonzentrationen) bestimmte man K_{M} und V_{MAX} durch nichtlinearen Fit auf die Gleichung$\text{V =} \frac{{\text{V}}_{\text{max}} \text{*[S]}}{{\text{[S]+K}}_{\text{M}}}$

Aus den geseamten Meßreihen mit 16 Datensätzen (Messungen bei 4 verschiedene Substratkonzentrationen und jeweils 4 verschiedenenen Inhibitorkonzentrationen) erhält man den Kᵢ-Wert durch nichtlineare Regression aus der Gleichung${\text{V}}_{\text{i}} \text{=} \frac{{\text{V}}_{\text{max}} \text{*[S]}}{{\text{K}}_{\text{M}} {\text{*(1+[I]/K}}_{\text{i}} \text{)+[S]}}$ wobei V_{Max} die maximale Geschwindigkeit in Abwesenheit eines Inhibitors, K_{M} die Michaliskonstante und [S] die Substratkonzentration bedeuten.

In Tabelle 1 sind die gemessenen Kᵢ-Werte in [µM] angegeben.

### FXa:

Stocklösung: 990 µl Phophatpuffer-Lösung (Herstellung siehe unten) wurden mit 10 µl humanem Faktor Xa (Boehringer Mannheim GmbH; 10 U; Suspension) versetzt und auf Eis maximal 4 Stunden gelagert. Zur Messung werden 850 µl Phosphatpuffer mit 100 µl Substrat [N-Methoxycarbonyl-(D)-norleucyl-glycyl-(L)-arginin-4-nitroanilin-Acetat; Chromozym X; Boehringer Mannheim GmbH; verwendete Substratkonzentrationen 800, 600, 400 und 200 µM; K_{M} 400 µM] und 25 µl Inhibitor-Lösung oder 25 µl DMSO (Kontrolle) im Photometer thermostatiert (25°C). Durch Zugabe von 25 µl Stocklösung wird die Reaktion gestartet.

### Thrombin:

Humanes α-Thrombin (Sigma; 100 U; spezifische Aktivität: 2000 NIH-units/mg) wird in 1 ml Wassser gelöst und in Portionen zu 20 µl bei - 18°C gelagert Stocklösung: 1480 µl Phophatpuffer-Lösung (Herstellung siehe unten) wurden mit 20 µl der wie voranstehend hergestellten α-Thrombin-Lösung versetzt und auf Eis maximal 4 Stunden gelagert. Zur Messung werden 850 µl Phosphatpuffer mit 100 µl Substrat [H-(D)-Phe-Pip-Arg-4-nitroanilin-dihydrochlorid; S-2238: Kabi; verwendete Substratkonzentrationen 100, 50, 30 und 20 µM; K_{M} 4 µM) und 25 µl Inhibitor-Lösung oder 25 µl DMSO (Kontrolle) im Photometer thermostatiert (25°C). Durch Zugabe von 25 µl Stocklösung wird die Reaktion gestartet.

### Trypsin:

10 mg bovines pankreatisches Trypsin (Sigma) wird in 100 ml 1 mM Salzsäure gelöst und im Kühlschrank bei 2-8°C aufbewahrt. Stocklösung: 990 µl 1 mM Salzsäure wurden mit 10 µl der wie voranstehend hergestellten Trypsin-Lösung versetzt und auf Eis maximal 4 Stunden gelagert. Zur Messung werden 850 µl Phosphatpuffer mit 100 µl Substrat [H-(D)-Phe-Pip-Arg-4-nitroanilin-dihydrochlorid; S-2238; Kabi; verwendete Substratkonzentrationen 100, 50, 30 und 20 µM; K_{M} 45 µM) und 25 µl Inhibitor-Lösung oder 25 µl DMSO (Kontrolle) im Photometer thermostatiert (25°C). Durch Zugabe von 25 µl Stocklösung wird die Reaktion gestartet.

### Herstellung der 0.1M Phosphatpuffer-Lösung (pH 7.5, 0.2 M NaCl):

8.90 g Na₂HPO₄ · 2 H₂O, 5.84 g NaCl und 2.50 g Polyethylenglykol 6000 werden in 400 ml destilliertem Wasser gelöst und mit destilliertem Wasser auf ein Gesamtvolumen von 500 ml aufgefüllt (Lösung I). 1.36 g KH₂PO₄, 1.17 g NaCl und 0.50 g Polyethylenglykol 6000 werden in 80 ml destilliertem Wasser gelöst und mit destilliertem Wasser auf ein Gesamtvolumen von 100 ml aufgefüllt (Lösung II). Dann gibt man soviel Lösung II (ca. 85 ml) zu Lösung I bis der pH-Wert 7.5 beträgt. Die Pufferlösung wird stets frisch hergestellt (bei Lagerung im Kühlschrank bei 4°C maximal 10 Tage haltbar).

**Tabelle 1**

| Pharmakologische Daten von Beispielverbindung 1 | | | | | | | | |
|---|---|---|---|---|---|---|---|---|
| Beispiel Nr. | Ki (fXa) | Ki (Thrombin) | Ki (Trypsin) | APTT 1000 | APTT 100 | APTT 10 | APTT 1 | TT 500 |
| **1** | 0.010 | 2 | 0.1 | > 300 | 140 | 50 | 12 | 40 |

## Patentansprüche

1. Verbindungen der allgemeinen Formel I 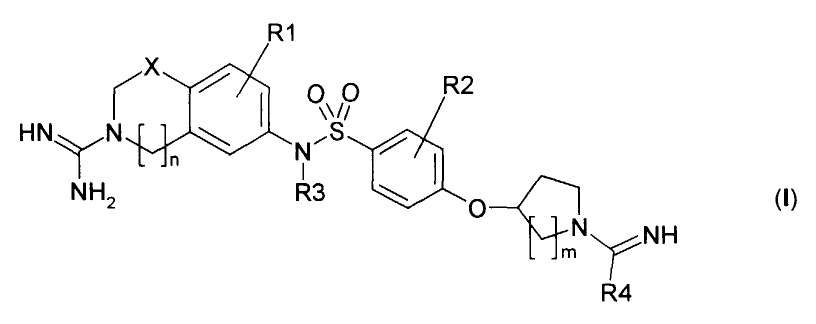 in der
R¹, R² unabhängig voneinander ein Wasserstoffatom, ein Halogenatom, eine Hydroxygruppe, eine Alkylgruppe, eine Cycloalkylgruppe, eine Alkenylgruppe, eine Alkinylgruppe, ein Arylrest, ein Heteroarylrest, eine Alkoxygruppe, eine Aralkyloxygruppe, eine Alkenyloxygruppe, eine Alkinyloxygruppe, eine Carboxylgruppe, eine Alkoxycarbonylgruppe, eine Alkenyloxycarbonylgruppe, eine Alkinyloxycarbonylgruppe, eine Hydroxyalkylgruppe, eine Alkoxyalkylgruppe, eine Carboxyalkylgruppe, eine Alkyloxycarbonylalkylgruppe, eine Alkenyloxycarbonylalkylgruppe oder eine Alkinyloxycarbonylalkylgruppe sein können;
R³ ein Wasserstoffatom, eine Alkylgruppe, eine Cycloalkylgruppe, eine Alkenylgruppe, eine Alkinylgruppe, einen Aralkylrest, eine Hydroxyalkylgruppe, eine Alkoxyalkylgruppe, eine Aminoalkylgruppe, eine Carboxyalkylgruppe, eine Alkyloxycarbonylalkylgruppe, eine Alkenyloxycarbonylalkylgruppe oder eine Alkinyloxycarbonylalkylgruppe, ein Alkylcarbonylrest, eine Arylcarbonylgruppe, eine Carboxyalkylsulfonylgruppe, eine Alkyloxycarbonylalkylsulfonylgruppe, eine Dihydroxyborylalkylgruppe, eine Dialkoxyborylalkylgruppe oder eine gegebenenfalls substituierte 1,3,2-Dioxaborolanylalkylgruppe oder eine gegebenenfalls substituierte 1,3,2-Dioxaborinanylalkylgruppe sein kann;
R⁴ eine gegebenenfalls substituierte Aminogruppe, eine Alkylgruppe, einen Cycloalkylrest, einen gegebenenfalls substituierten Arylrest oder einen gegebenenfalls substituierten Heteroarylrest bedeutet;
X eine Einfachbindung, eine Carbonylgruppe eine Alkylen- oder eine Alkylenoxygruppe bedeutet;
n die Zahl 1 oder 2 bedeutet und
m eine ganze Zahl zwischen 1 und 4 bedeutet,
sowie deren Hydrate, Solvate und physiologisch verträgliche Salze, optisch aktive Formen, Racemate und Diastereomerengemische.

2. Verbindungen gemäß Anspruch 1, in denen
R¹, R² gleich oder verschieden sind und ein Wasserstoffatom, ein Fluoratom, ein Chloratom, eine Hydroxygruppe, eine Methylgruppe, eine Ethylgruppe, eine Methoxygruppe, eine Benzyloxygruppe, eine Allyloxygruppe, eine Carboxylgruppe, eine Methoxy- oder Ethoxycarbonylgruppe, eine Hydroxymethyl- oder Hydroxyethylgruppe oder eine Carboxymethylgruppe bedeuten;
R³ ein Wasserstoffatom, eine Methylgruppe, eine Ethylgruppe, eine Isopropylgruppe, eine Cyclopropylgruppe, eine Cyclohexylgruppe, eine Allylgruppe, eine Propargylgruppe, eine Benzylgruppe, eine Hydroxyethylgruppe, eine Hydroxypropylgruppe, eine Methoxyethylgruppe, eine Aminoethylgruppe, eine Carboxymethylgruppe, eine Carboxyethylgruppe, eine Carboxypropylgruppe, eine Ethoxycarbonylmethylgruppe, eine Ethoxycarbonylethylgruppe, eine Ethoxycarbonylpropylgruppe, eine Acetylgruppe, eine 4-Methoxybenzoylgruppe, eine Carboxymethylsulfonylgruppe, eine Carboxyethylsulfonylgruppe, eine Carboxypropylsulfonylgruppe, eine Ethoxycarbonylmethylsulfonylgruppe, eine Ethoxycarbonylethylsulfonylgruppe, eine Ethoxycarbonylpropylsulfonylgruppe, eine Dihydroxyborylmethylgruppe, eine Dihydroxyborylpropylgruppe, eine 4,4,5,5-Tetramethyl-1,3,2-dioxaborolanylmethylgruppe bedeutet;
R⁴ eine Aminogruppe, eine Methylgruppe, eine Ethylgruppe, eine Cyclopropylgruppe, eine Cyclohexylgruppe, eine 4-Methoxyphenylgruppe oder eine Thienylgruppierung bedeutet;
X eine Methylengruppe bedeutet;
n die Zahl 1 bedeutet und
m die Zahl 1 oder die Zahl 2 sein kann.
sowie deren Hydrate, Solvate und physiologisch verträgliche Salze, optisch aktive Formen, Racemate und Diastereomerengemische.

3. Pharmazeutische Zubereitungen, enthaltend mindestens eine Verbindung der Formel I gemäß Anspruch 1 oder 2 neben üblichen Träger- und Hilfsstoffen.

4. Verwendung von Verbindungen der Formel I gemäß Anspruch 1 oder 2 zur Herstellung von Arzneimitteln mit antithromboembolischer Wirkung.
